# EUROPEAN PATENT SPECIFICATION

(11) **EP 3 036 782 B1**
(45) Date of publication and mention of the grant of the patent: **09.10.2019**
(21) Application number: 14750740.4
(22) Date of filing: 14.08.2014
(51) Int. Cl.: H01L 51/46, H01L 27/30

(54) **NEW ABSORBER FOR ORGANIC HETEROJUNCTION SOLAR CELLS**
NEUE ABSORBER FÜR ORGANISCHE HETEROVERBINDUNGS-SOLARZELLEN
NOUVEL ABSORBEUR POUR CELLULES SOLAIRES ORGANIQUES À HÉTÉROJONCTION

(30) Priority: 23.08.2013 EP 13181536
(43) Date of publication of application: 29.06.2016
(73) Proprietor: BASF SE, 67056 Ludwigshafen am Rhein (DE)
(72) Inventor: TANABE, Junichi, Takarazuka-shi Hyogo 665-0847 (JP); NISHIMAE, Yuichi, Ibaraki-Shi Osaka 567-0887 (JP); EICKHOFF, Christian, 68259 Mannheim (DE); ERK, Peter, 67227 Frankenthal (DE); SENS, Rüdiger, 67069 Ludwigshafen (DE); SEND, Robert, 76137 Karlsruhe (DE); SUNDARRAJ, Sudhakar, Singapore 120711 (SG)
(86) International application number: PCT/EP2014/067396
(87) International publication number: WO 2015/024851

(56) References cited:
- EP-A2- 0 887 817
- WO-A2-2007/020442
- JP-A- 2007 026 930
- QIAO F ET AL: "Enhanced photovoltaic characteristics of solar cells based on n-type triphenodioxazine derivative", MICROELECTRONICS JOURNAL, MACKINTOSH PUBLICATIONS LTD. LUTON, GB, vol. 39, no. 12, 16 May 2008 (2008-05-16), pages 1568-1571, XP025678419, ISSN: 0026-2692, DOI: 10.1016/J.MEJO.2008.02.027 [retrieved on 2008-11-18]
- HAIYAN LI ET AL: "Tetraazabenzodifluoranthene Diimides: Building Blocks for Solution-Processable n-Type Organic Semiconductors", ANGEWANDTE CHEMIE INTERNATIONAL EDITION, vol. 52, no. 21, 17 May 2013 (2013-05-17), pages 5513-5517, XP55088185, ISSN: 1433-7851, DOI: 10.1002/anie.201210085
- YUECHAO WU ET AL: "Crystal Structure and Phototransistor Behavior of N-Substituted Heptacence", ACS APPLIED MATERIALS & INTERFACES, vol. 4, no. 4, 25 April 2012 (2012-04-25), pages 1883-1886, XP55147002, ISSN: 1944-8244, DOI: 10.1021/am3003389

## Description

### Field of the invention

The present invention relates to a photoactive material comprising a donor substance and an acceptor substance, wherein the donor substance comprises or consists of one or more compounds of formula (I) described below, or the acceptor substance comprises or consists of one or more compounds of formula (I) described below, or the donor substance comprises or consists of a first compound of formula (I) described below and the acceptor substance comprises a second compound of formula (I) described below with the proviso that the first and second compound are not the same, as well as to an organic solar cell or photodetector comprising said photoactive material. The present invention also relates to a photoelectric conversion device comprising or consisting of two or more organic solar cells comprising said photoactive material and to compounds of formula (I) as described below for use as donor substance or as acceptor substance in a photoactive material.

### Description of the Prior Art

The increasing global energy demand and the growing concerns for a clean and sustainable energy solution for the future has made people to look into energy generation from renewable sources with much interest. Owing to diminishing fossil raw materials and the CO₂ which is formed in the combustion of these raw materials and is active as a greenhouse gas, direct energy generation from sunlight is playing an increasing role. Of all the renewable energy sources that are available for use, photovoltaic or the process of converting sunlight into electricity assumes paramount importance, due to the fact that the sun gives us abundant amount of energy, much more than what is needed to meet our present and future energy requirements.

"Photovoltaic" is understood to mean the direct conversion of radiative energy, principally solar energy, to electrical energy. The conventional way of harnessing sunlight is by using solar cells typically made of silicon (in the form of single crystalline, polycrystalline or amorphous silicon) or by the use of thin film technologies like CdTe, CIGS, etc. Factors such as high cost for the energy conversion process, limited availability of raw materials and toxic nature of some of the materials used like Cd, prevents their widespread use in harnessing the tremendous potential of the sun.

One promising alternative approach for solar energy conversion is the use of organic carbon based materials instead of the conventional inorganic materials used in the photovoltaic industry, Carbon based materials or the so called organic dyes that could be used for this purpose have certain advantages over conventional inorganics, for example organic dyes being strong light absorbers can absorb light more efficiently than their inorganic counterparts. As a consequence of this only a small amount of material is required for making solar cells. Further they are easy to process; thin layers of organic dyes can easily be formed by wet printing or thermal evaporation process.

In contrast to inorganic solar cells, in organic solar cells the light does not directly generate free charge carriers in organic solar cells, but rather excitons are formed first, i.e. electrically neutral excited states in the form of electron-hole pairs. These excitons can be separated only by very high electric fields or at suitable interfaces.

In organic solar cells, sufficiently high fields are unavailable, and so all existing concepts for organic solar cells are based on exciton separation at photoactive interfaces (organic donor-acceptor interfaces or interfaces to an inorganic semiconductor). For this purpose, it is necessary that excitons which have been generated in the volume of the organic material can diffuse to this photoactive interface. The diffusion of excitons to the active interface thus plays a critical role in organic solar cells. In order to make a contribution to the photocurrent, the exciton diffusion length in a good organic solar cell must at least be in the order of magnitude of the typical penetration depth of light, in order that the predominant portion of the light can be utilized. The efficiency of an organic solar cell is characterized by its open-circuit voltage V_{oc}. Further important characteristics are the short-circuit current I_{sc}, the fill factor FF and the resulting efficiency η. The first organic solar cell with an efficiency in the percent range was described by Tang et al. in 1986 (CW. Tang et al., Appl. Phys. Lett. 1986, 48, 183). It consisted of a two-layer system with copper phthalocyanine (CuPc) as the donor substance (p-semiconductor) and perylene-3,4,9,10-tetracarboxylic acid bisimidazole (PTCBI) as the acceptor substance (n-semiconductor).

A current aim in organic photovoltaic is to provide a new generation of solar cells which are significantly less expensive than solar cells composed of silicon or other inorganic semiconductors such as cadmium indium selenide or cadmium telluride. For this purpose, there is additionally a need for suitable semiconductive light-absorbing materials. One means of absorbing a large amount of light and of achieving good efficiencies is to use a pair of semiconductor materials which are complementary with regard to light absorption, for example comprising a short-wave-absorbing n-semiconductor and a long-wave-absorbing p-semiconductor. This concept is also the basis of the aforementioned first organic solar cell, known as the Tang cell.

Even though many fullerene compounds absorb the light only weakly, it has been found that efficient solar cells can be produced when fullerenes or fullerene derivatives, such as C₆₀ or C₇₂, are used as n-semiconductors. It is additionally known, when using weakly absorbing semiconductor materials, to build two solar cells one on top of another. In that case, one cell comprises a combination of the weakly absorbing semiconductor with a semiconductor complementary thereto, which absorbs the short-wave radiation, and the other cell a combination of the weakly absorbing semiconductor with a semiconductor complementary thereto, which absorbs the long-wave radiation. For such tandem cells for combination with fullerenes or fullerene derivatives, two suitable p-semiconductors are required, one of which absorbs the short-wave radiation and one the long-wave radiation.

The discovery of suitable semiconductor combinations is not trivial. In tandem cells, the open-circuit voltages V_{oc} of the individual components are additive. The total current is limited by the component cell with the lowest short-circuit current I_{sc}. The two semiconductor materials of the individual cells thus have to be adjusted exactly with respect to one another. There is therefore a great need for p-semiconductive organic absorber materials with long-wave absorption for use in organic solar cells in combination with fullerenes or fullerene derivatives, and especially in tandem cells, with high open-circuit voltage and acceptable short-circuit current.

In light of this, there has been an ever growing interest in the scientific community to look for alternate materials for light energy conversion. Thus, there has been a continued search for new materials which includes both organic (semiconducting) small molecules and polymers. Compared to conjugated polymers, small molecular semiconductors offer several intrinsic advantages in organic solar cell applications. They are monodisperse with a defined molecular structure and are also easy to synthesize and purify. The quest for new small organic semiconductor molecules has led to the investigation of several organic dye classes as donor materials, which includes phthalocyanines, porphyrins, merocyanines, organic acenes, oligothiophenes, squarines, rylenes, hexabenzo-coronene, BODIPY dyes, etc. These donor or p-type semiconducting absorber materials can be further sub-classified based on their structure *viz*, Donor (D), Donor-Acceptor-Donor (D-A-D), Acceptor-Donor-Acceptor (A-D-A), Donor-Acceptor-Donor-Acceptor-Donor (D-A-D-A-D) and Acceptor-Donor-Acceptor-Donor-Acceptor (A-D-A-D-A) where "D" represent an electron rich donor segment and "A" represent an electron deficient acceptor segment. By carefully choosing the donor and acceptor segments, it is possible to tune the electronic as well as the photophysical properties of these molecules.

A detailed summary of various organic small molecules and also the device structures that has been investigated for use in organic solar cells can be found in published literatures, e.g. "Small molecule semiconductors for high-efficiency organic photovoltaics", Yuze Lin, Yongfang Lia and Xiaowei Zhan; Chem. Soc. Rev. 2012, 41, 4245-4272, "Small molecule organic semiconductors on the move: Promises for future solar energy technology", Amaresh Mishra and Peter Baeuerle; Angew. Chem. Int. Ed. 2012, 51, 2020-2067, "Small-molecule solar cells - Status and perspectives", M. Riede, T. Mueller, W Tress, R. Schueppel and K. Leo; Nanotechnology 2008, 19, 424001.

It has now been found that, surprisingly, compounds of formula (I) defined below are advantageously suitable as electron donors (p-semiconductors, hole conductors) in organic photovoltaics. They are especially suitable for a combination with at least one fullerene compound, such as C₆₀, as an electron acceptor (n-semiconductor, electron conductor). It has especially been found that compounds of formula (I) are suitable for use in tandem cells, since they have a long-wave absorption and exhibit a high open-circuit voltage in combination with a fullerene compound, such as C₆₀. Solar cells comprising a photoactive material comprising a compound of formula (I) show improved efficiencies.

Qiao et al. (F. Qiao, A. Liu, Y. Xiao, Y. P. Ou, J. Q. Zhang, Y. C. Sang, Microelectron. J. 2008, 39, 1568-1571.) disclose an organic solar cell with an active layer with a mixture of poly (2-methoxy-5-(2'-ethyl-hexyloxy)-1, 4-phenylene vinylene) (MEH-PPV) : 1-(3-methoxycarbonyl)-propyl-1-1-phenyl-(6,6)C61 (PCBM) 3, 10-di(trifluoromethane) triphenodioxazine (TFTD), wherein PCBM is acting as acceptor and MEH-PPV and TFTD are acting as donor.

EP 0 887 817 A2 discloses a dye sensitized solar cell employing a phthalocyanine derivative, a dioxazine derivative or an indigo derivative as sensitizer.

Li et al. (H. Li, F. S. Kim, G. Ren, E. C. Hollenbeck, S. Subramaniyan, S. A. Jenekhe, Angew. Chem. Int. Ed. 2013, 52, 5513-5517.) teach the synthesis of tetraazabenzodifluoranthene diimides (BFIs), which were used as acceptor material in polymer solar cells.

JP 2007 026930 A discloses a dye sensitized solar cell, which has a latent pigment as at least one kind of dyes, resulting in high photoelectric conversion efficiency and excellent stability of the solar cell.

Wu et al. (Y. Wu, Z.. Yin, J. Xiao, Y. Liu, F. Wei, K. J. Tan, C. Kloc, L. Huang, Q. Yan, F. Hu, H. Zhang, Q. Zhang, ACS Appl. Mater. Interfaces, 2012, 4, 1883-1886.) disclose a phototransistor device based on 6,8,15,17-Tetraaza-1.18,4.5,9.10,13.14-tetrabenzoheptacene (TTH).

### Summary of the invention

The subject-matter of the present invention is defined in the attached claims.

According to a first aspect of the present invention, a photoactive material is provided comprising a donor substance and an acceptor substance, wherein
**a.** the donor substance comprises or consists of one or more compounds of formula (I), or
**b.** the acceptor substance comprises or consists of one or more compounds of formula (I), or
**c.** the donor substance comprises or consists of a first compound of formula (I) and the acceptor substance comprises a second compound of formula (I) with the proviso that the first and second compound are not the same, 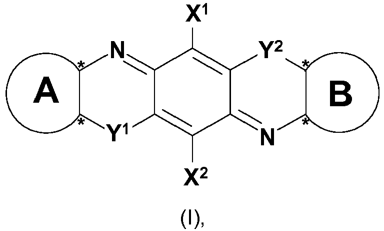 wherein in formula (I)
   each of A and B independently of each other indicates a 5- or 6-membered ring which includes the carbon atoms marked with *, respectively,
   wherein each of the rings A and B is independently of each other
      (i) aromatic or heteroaromatic,
      (ii) unsubstituted or substituted,
         and is
      (iii) not annealed or annealed with one or more further rings not including carbon atoms marked with *, respectively, or is connected with one or more further rings by a single common atom to give a spiro compound,
   X¹, X² are independently of each other selected from the group consisting of hydrogen and substituents,
   Y¹, Y² are independently of each other selected from the group consisting of O, S, NR¹, PR¹, Se and Te,
   wherein
   each R¹ is independently selected from the group consisting of H, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl,
      characterised in that in formula (I) A or B or each of A and B independently of each other indicates a 5 or 6-membered ring present in a moiety selected from the group consisting of 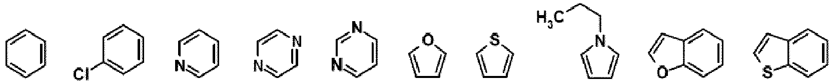 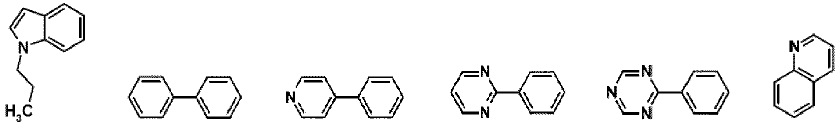 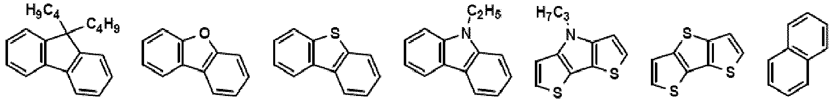 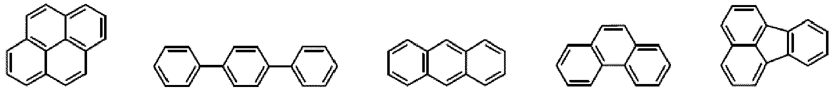 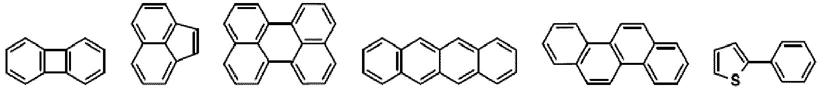 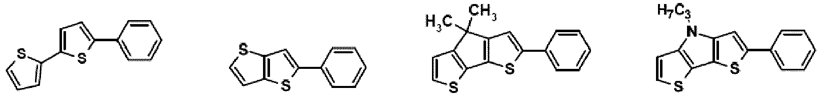 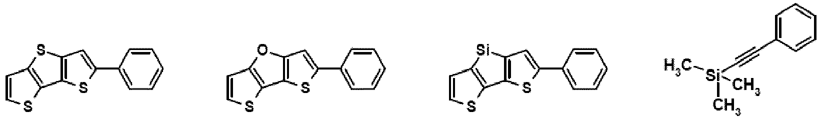 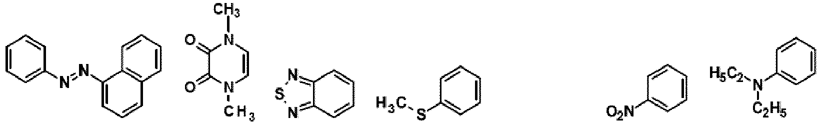 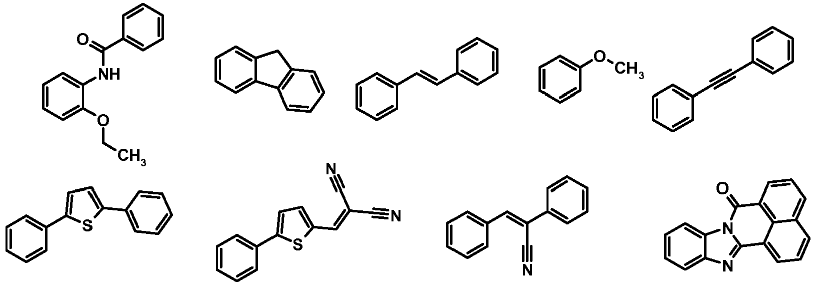 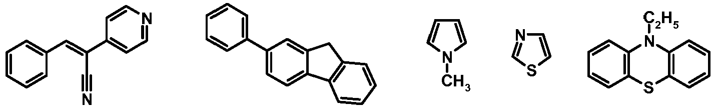
      wherein in each 5 or 6-membered ring present in said moieties and independently of each other indicating A or B, respectively, two adjacent carbon atoms are identical with carbon atoms marked with * in formula (I),
      wherein the donor substance and the acceptor substance are part of respective layers of a donor-acceptor bilayer or part of a donor-acceptor mixed layer.

According to another aspect of the invention, an organic solar cell or photodetector is provided comprising a photoactive material according to the present invention, as defined above.

In a further aspect, the present invention relates to a photoelectric conversion device comprising or consisting of two or more organic solar cells according to the present invention, as defined above, wherein the organic solar cells are preferably arranged as tandem cells (multi-junction solar cells) or as inverted tandem cells.

In yet a further aspect, the present invention relates to a compound of formula (I) as defined above for use as donor substance or as acceptor substance in a photoactive material, wherein the photoactive material is part of a layer of a donor-acceptor bilayer or part of a donor-acceptor mixed layer (bulk heterojunction, BHJ).

### Description of figures

Figure 1 shows a solar cell having normal structure.
Figure 2 shows a solar cell with inverse structure.
Figure 3 shows the structure of a solar cell with normal structure and with a donor- acceptor interface in the form of a bulk heterojunction.
Figure 4 shows the structure of a solar cell with inverse structure and with a donor- acceptor interface in the form of a bulk heterojunction.
Figure 5 shows the structure of a tandem cell.
Figure 6 shows the current density-versus-voltage (J-V) curve for an inverted BHJ-cell according to the present invention.
Figure 7 shows the absorption spectrum and the External Quantum Efficiency (EQE) spectrum of compound 1 according to formula (I).
Figure 8 shows an inverted BHJ-cell device setup according to the invention.
Figure 9 shows the current density-versus-voltage (J-V) curve, the absorption spectrum and the External Quantum Efficiency (EQE) spectrum of compound 26 for an inverted BHJ-cell according to the present invention.
Figure 10 shows the current density-versus-voltage (J-V) curve, the absorption spectrum and the External Quantum Efficiency (EQE) spectrum of compound 27 for an inverted BHJ-cell according to the present invention.
Figure 11 shows the current density-versus-voltage (J-V) curve, the absorption spectrum and the External Quantum Efficiency (EQE) spectrum of compound 79 for an inverted BHJ-cell according to the present invention.

### Detailed description of the invention

In the context of the invention, the expression "photoactive material" represents a material having a photoactive heterojunction formed by at least one hole-conducting organic substance (donor substance, p-semiconductor) and at least one electron-conducting organic substance (acceptor substance, n-semiconductor).

In the context of the present application, an organic substance is referred to as "hole-conducting" when the charge carriers which are formed as a result of light absorption and charge separation at a heterojunction ("photogenerated charge carriers") are transported within the material in the form of holes. Accordingly, an organic substance is referred to as "electron-conducting" when photogenerated charge carriers are transported within the material in the form of electrons.

A "heterojunction" refers to an interface region between the electron-conducting and the hole-conducting substance.

A "photoactive heterojunction" refers to a heterojunction between the electron-conducting and the hole-conducting substance when excited states formed by light absorption in the electron-conducting and/or the hole-conducting substance ("excitons"), which are separated in the region of the heterojunction into the individual charge carriers, namely electrons and holes, which are then in turn transported through the electron-conducting substance/the hole-conducting substance to electrical contacts, where electrical energy can be drawn off.

A "flat heterojunction" refers to a heterojunction between the electron-conducting and the hole-conducting substance when the interface between the electron-conducting and the hole-conducting substance is formed as an essentially cohesive surface between the two substance layers, namely one layer of the electron-conducting substance and one layer of the hole-conducting substance, i.e. a bilayer configuration (*cf.* C. W. Tang, Appl. Phys. Lett. 1986, 48 (2), 183-185 or N. Karl et al., Mol. Cryst. Liq. Cryst. 1994, 252, 243-258).

A "bulk heterojunction" refers to a heterojunction between the electron-conducting and the hole-conducting substance when the electron-conducting substance and the hole-conducting substance are at least partly mixed with one another, such that the interface between the electron-conducting and the hole-conducting substance comprises a multitude of interface sections distributed over the volume of the substance mixture (*cf*. C.J. Brabec et al., Adv. Funct. Mater. 2001, 11(1), 15).

In the context of the present invention, the expression "alkyl" comprises straight-chain or branched alkyl. Alkyl preferably has 1 to 24 carbon atoms, more preferably 2 to 12 carbon atoms and most preferably 3 to 8 carbon atoms. Examples of alkyl groups are methyl, ethyl, n-propyl, isopropyl, n-butyl, isobutyl, sec-butyl, tert-butyl, n-pentyl, n-hexyl, n-heptyl, n-octyl, n-nonyl, n-decyl, n-undecyl, n-dodecyl, n-tridecyl, n-tetradecyl, n-hexadecyl, n-octadecyl and n-eicosyl.

Substituted alkyl groups may, depending on the length of the alkyl chain, have one or more (e.g. 1, 2, 3, 4, 5 or more than 5) substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, carboxylate, alkylcarbonyloxy, carbamoyl, sulfonate, sulfamino, sulfamide, amidino. Cycloalkyl, heterocycloalkyl, aryl and heteroaryl substituents of the alkyl groups may in turn be unsubstituted or substituted; suitable substituents are the substituents mentioned above for these groups.

The above remarks regarding unsubstituted and substituted alkyl also apply to unsubstituted and substituted alkenyl as well as unsubstituted and substituted alkynyl.

In the context of the invention, "cycloalkyl" denotes a cycloaliphatic group preferably having 3 to 8 carbon atoms, more preferably 5 or 6 carbon atoms. Examples of cycloalkyl groups are cyclopropyl, cyclobutyl, cyclopentyl, cyclohexyl, cycloheptyl and cyclooctyl.

Substituted cycloalkyl groups may, depending on the ring size, have one or more (e.g. 1, 2, 3, 4, 5 or more than 5) substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, carboxylate, alkylcarbonyloxy, carbamoyl, sulfonate, sulfamino, sulfamide, amidino.

In the case of substitution, the cycloalkyl groups preferably bear one or more (e.g. 1, 2, 3, 4, 5 or more than 5) alkyl groups. Examples of substituted cycloalkyl groups are 2- and 3-methyl-cyclopentyl, 2- and 3-ethylcyclopentyl, 2-, 3- and 4-methylcyclohexyl, 2-, 3- and 4-ethylcyclohexyl, 2-, 3- and 4-propylcyclohexyl, 2-, 3- and 4-isopropylcyclohexyl, 2-, 3- and 4-butylcyclohexyl, 2-, 3- and 4-sec-butylcyclohexyl, 2-, 3- and 4-tert-butylcyclohexyl, 2-, 3- and 4-methylcycloheptyl, 2-, 3- and 4-ethylcycloheptyl, 2-, 3- and 4-propylcycloheptyl, 2-, 3- and 4-isopropylcycloheptyl, 2-, 3- and 4-butylcycloheptyl, 2-, 3- and 4-sec-butylcycloheptyl, 2-, 3- and 4-tert-butylcycloheptyl, 2-, 3-, 4- and 5-methyl-cyclooctyl, 2-, 3-, 4- and 5-ethylcyclooctyl, 2-, 3-, 4- and 5-propylcyclooctyl.

In the context of the present invention, the expression "aryl" comprises mono- and polycyclic aromatic hydrocarbon groups preferably having 5 to 30 carbon atoms, more preferably 6 to 14 carbon atoms, most preferably 6 to 10 carbon atoms. Examples of aryl groups are phenyl, naphthyl, indenyl, fluorenyl, anthracenyl, phenanthrenyl, naphthacenyl, chrysenyl and pyrenyl.

Substituted aryls may, depending on the number and size of their ring systems, have one or more (e.g. 1, 2, 3, 4, 5 or more than 5) substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, carboxylate, alkylcarbonyloxy, carbamoyl, sulfonate, sulfamino, sulfamide, amidino. Cycloalkyl, heterocycloalkyl, aryl and heteroaryl substituents of the aryl groups may in turn be unsubstituted or substituted; suitable substituents are the substituents mentioned above for these groups.

In case of substitution, the aryl groups preferably bear one or more (e.g. 1, 2, 3, 4, 5 or more than 5) alkyl groups. Examples of substituted aryl groups are 2-, 3- and 4-methylphenyl, 2,4-, 2,5-, 3,5- and 2,6-dimethylphenyl, 2,4,6-trimethylphenyl, 2-, 3- and 4-ethylphenyl, 2,4-, 2,5-, 3,5- and 2,6-diethylphenyl, 2,4,6-triethylphenyl, 2-, 3- and 4-propylphenyl, 2,4-, 2.5- , 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3- and 4-sec-butylphenyl, 2,4-, 2,5-, 3,5- and 2,6-di-sec-butylphenyl, 2,4,6-tri-sec-butylphenyl, 2-, 3- and 4-tert-butylphenyl, 2,4-, 2,5-, 3,5- and 2,6-di-tert-butylphenyl, 2,4,6-tri-tert-butylphenyl.

In the context of the present invention, the expression "heterocycloalkyl" comprises nonaromatic, unsaturated or fully saturated, cycloaliphatic groups preferably having 3 to 8 ring atoms, more preferably 5 or 6 ring atoms. In the heterocycloalkyl groups 1, 2, 3, 4 or more than 4 of the ring carbon atoms are replaced by heteroatoms or heteroatom-containing groups. The heteroatoms or heteroatom-containing groups are preferably selected from -O-, -S- and -NR-, wherein R is preferably hydrogen, alkyl, cycloalkyl, heterocycloalkyl, aryl or heteroaryl. Heterocycloalkyl is unsubstituted or optionally bears one or more (e.g. 1, 2, 3, 4, 5, 6 or 7) identical or different substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, carboxylate, alkylcarbonyloxy, carbamoyl, sulfonate, sulfamino, sulfamide, amidino.

Examples of heterocycloalkyl groups are pyrrolidinyl, piperidinyl, 2,2,6,6-tetramethylpiperidinyl, imidazolidinyl, pyrazolidinyl, oxazolidinyl, morpholidinyl, thiazolidinyl, isothiazolidinyl, isoxazolidinyl, piperazinyl, tetrahydrothiophenyl, dihydrothien-2-yl, tetrahydrofuranyl, dihydrofuran-2-yl, tetrahydropyranyl, 1,2-oxazolin- 5-yl, 1,3-oxazolin-2-yl and dioxanyl.

Substituted heterocycloalkyl groups may, depending on the ring size, have one or more (e.g. 1, 2, 3, 4, 5 or more than 5) substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, carboxylate, alkylcarbonyloxy, carbamoyl, sulfonate, sulfamino, sulfamide, amidino.

In the context of the present invention, the expression "heteroaryl" comprises heteroaromatic, mono- or polycyclic groups. In addition to the ring carbon atoms, these groups have 1, 2, 3, 4 or more than 4 ring heteroatoms. The heteroatoms are preferably selected from oxygen, nitrogen, selenium and sulfur. The heteroaryl groups have preferably 5 to 28, more preferably 6 to 14, ring atoms.

Monocyclic heteroaryl groups are preferably 5- or 6-membered heteroaryl groups, such as 2-furyl (furan-2-yl), 3-furyl (furan-3-yl), 2-thienyl (thiophen-2-yl), 3-thienyl (thiophen-3-yl), selenophen-2-yl, selenophen-3-yl, 1H-pyrrol-2-yl, 1H-pyrrol-3-yl, pyrrol-1-yl, imidazol-2-yl, imidazol-1-yl, imidazol-4-yl, pyrazol-1-yl, pyrazol-3-yl, pyrazol-4-yl, pyrazol-5-yl, 3-isoxazolyl, 4-isoxazolyl, 5-isoxazolyl, 3-isothiazolyl, 4-isothiazolyl, 5-isothiazolyl, 2-oxazolyl, 4-oxazolyl, 5-oxazolyl, 2-thiazolyl, 4-thiazolyl, 5-thiazolyl, 1,2,4-oxadiazol-3-yl, 1,2,4-oxadiazol-5-yl, 1,3,4-oxadiazol-2-yl, 1,2,4-thiadiazol-3-yl, 1,2,4-thiadiazol-5-yl, 1,3,4-thiadiazol-2-yl, 4H-[1,2,4]-triazol-3-yl, 1,3,4-triazol-2-yl, 1,2,3-triazol-1-yl, 1,2,4- triazol-1-yl, pyridin-2-yl, pyridin-3-yl, pyridin-4-yl, 3-pyridazinyl, 4-pyridazinyl, 2-pyrimidinyl, 4-pyrimidinyl, 5-pyrimidinyl, 2-pyrazinyl, 1,3,5-triazin-2-yl and 1,2,4-triazin- 3-yl.

Polycyclic heteroaryl groups have 2, 3, 4 or more than 4 fused rings. The fused-on rings may be aromatic, saturated or partly unsaturated. Examples of polycyclic heteroaryl groups are quinolinyl, isoquinolinyl, indolyl, isoindolyl, indolizinyl, benzofuranyl, isobenzofuranyl, benzothiophenyl, benzoxazolyl, benzisoxazolyl, benzthiazolyl, benzoxadiazolyl; benzothiadiazolyl, benzoxazinyl, benzopyrazolyl, benzimidazolyl, benzotriazolyl, benzotriazinyl, benzoselenophenyl, thienothiophenyl, thienopyrimidyl, thiazolothiazolyl, dibenzopyrrolyl (carbazolyl), dibenzofuranyl, dibenzothiophenyl, naphtho[2,3-b]thiophenyl, naphtha[2,3-b]furyl, dihydroindolyl, dihydroindolizinyl, dihydroisoindolyl, dihydroquinolinyl, dihydroisoquinolinyl.

Substituted heteroaryl groups may, depending on the number and size of their ring systems, have one or more (e.g. 1, 2, 3, 4, 5 or more than 5) substituents. These are preferably each independently selected from cycloalkyl, heterocycloalkyl, aryl, heteroaryl, fluorine, chlorine, bromine, hydroxyl, mercapto, cyano, nitro, nitroso, formyl, acyl, carboxylate, alkylcarbonyloxy, carbamoyl, sulfonate, sulfamino, sulfamide, amidino.

According to a first aspect of the present invention, a photoactive material is provided comprising a donor substance and an acceptor substance, wherein
**a.** the donor substance comprises or consists of one or more compounds of formula (I), or
**b.** the acceptor substance comprises or consists of one or more compounds of formula (I), or
**c.** the donor substance comprises or consists of a first compound of formula (I) and the acceptor substance comprises a second compound of formula (I) with the proviso that the first and second compound are not the same, 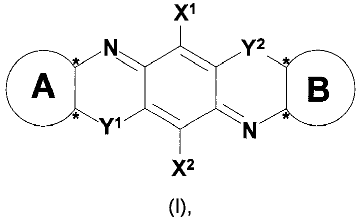 wherein in formula (I)
   each of A and B independently of each other indicates a 5- or 6-membered ring which includes the carbon atoms marked with *, respectively,
   wherein each of the rings A and B is independently of each other
      (i) aromatic or heteroaromatic,
      (ii) unsubstituted or substituted,
         and is
      (iii) not annealed or annealed with one or more further rings not including carbon atoms marked with *, respectively, or is connected with one or more further rings by a single common atom to give a spiro compound,
   X¹, X² are independently of each other selected from the group consisting of hydrogen and substituents,
   Y¹, Y² are independently of each other selected from the group consisting of O, S, NR¹, PR¹, Se and Te,
   wherein
   each R¹ is independently selected from the group consisting of H, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl.

In the context of the present invention option **a.** described above is preferred, i.e. it is preferred that in the inventive photoactive material the donor substance comprises or consists of one or more compounds of formula (I).

According to the present invention the donor substance and the acceptor substance of the inventive photoactive material are part of respective layers of a donor-acceptor bilayer or part of a donor-acceptor mixed layer (bulk heterojunction, BHJ).

According to the present invention it is preferred that in the compound of formula (I) each of the rings A and B is independently of each other (iii) annealed with one or more further unsubstituted or substituted rings not carbon atoms marked with *, respectively, wherein each of said one or more further rings is independently of each other non-aromatic, aromatic or heteroaromatic.

According to the present invention it is preferred that in the compound of formula (I) X¹, X² are independently of each other selected from the group consisting of H, D, F, Cl, Br, I, NO₂, CN, OH, C(O)OR¹, OC(O)OR¹, NR¹C(O)NR²R³, C(O)NR²R³, S(O)R¹, SO₂R¹, SO₃R¹, OSO₃R¹, COR¹, SiR¹R²R³, P(O)R¹R², P(O)OR¹OR², OR¹, SR¹, NR²R³, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl,
wherein
(i) R¹, R², R³ are independently of each other selected from the group consisting of H, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl;
   or
(ii) R¹ has the meaning as defined under (i), and
   R² and R³ together and in combination with the atom connecting R² and R³ constitute an aromatic or heteroaromatic or aliphatic 5 to 8-membered ring.

According to the present invention it is even more preferred that that in the compound of formula (I) X¹, X² are independently of each other selected from the group consisting of H, F, Cl, Br, I, NO₂, CN, C(O)OR¹, C(O)NR²R³, SiR¹R²R³, unsubstituted alkyl having 1 to 12 carbon atoms, substituted alkyl having 1 to 12 carbon atoms including substituents, unsubstituted aryl having 6 to 18 carbon atoms, substituted aryl having 6 to 18 carbon atoms including substituents, unsubstituted heteroaryl having 4 to 12 carbon atoms, and substituted heteroaryl having 4 to 12 carbon atoms including substituents.

According to the present invention it is also preferred that in the compound of formula (I) A and B are independently selected from the group consisting of unsubstituted aryl having 5 to 30 carbon atoms, substituted aryl having 5 to 30 carbon atoms including substituents, unsubstituted heteroaryl having 5 to 28 carbon atoms, substituted heteroaryl having 5 to 28 carbon atoms including substituents; wherein any substituent, if present, is a substituent Z¹, where Z¹ is independently of each other selected from the group consisting of unsubstituted alkyl having 1 to 24 carbon atoms, substituted alkyl having 1 to 24 carbon atoms including substituents, unsubstituted cycloalkyl having 3 to 8 carbon atoms, substituted cycloalkyl having 3 to 8 carbon atoms including substituents, F, Cl, Br, I, OR⁴, SR⁴, NR⁵R⁶, CN, NO₂, C(O)R⁷, N=N-R⁵, SiR⁸R⁹R¹⁰, C≡C-R¹¹, unsubstituted aryl having 6 to 20 carbon atoms, substituted aryl having 6 to 20 carbon atoms including substituents, unsubstituted heteroaryl having 4 to 12 carbon atoms, and W;
wherein R⁴, R⁵, R⁶ are independently of each other selected from the group consisting of H, alkyl having 1 to 8 carbon atoms, and aryl having 6 to 10 carbon atoms;
R⁷, R⁸, R⁹, R¹⁰ are independently of each other selected from the group consisting of H, OH, alkyl having 1 to 6 carbon atoms, aryl having 6 to 10 carbon atoms, O-alkyl having 1 to 8 carbon atoms, and O-aryl having 6 to 10 carbon atoms;
R¹¹ is independently of each other selected from the group consisting of aryl having 6 to 10 carbon atoms, heteroaryl having 4 to 10 carbon atoms, Si(alkyl having 1 to 6 carbon atoms)₃, and SiPh₃;
W is 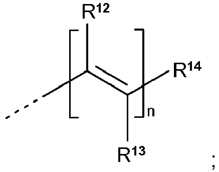
wherein n is selected from the group consisting of 1, 2, 3 and 4;
R¹², R¹³, R¹⁴ are independently of each other selected from the group consisting of H, CN, alkyl having from 1 to 8 carbon atoms, aryl having from 6 to 10 carbon atoms, and heteroaryl having from 4 to 10 carbon atoms.

According to the present invention it is especially preferred that in the compound of formula (I) X¹, X² are independently of each other selected from the group consisting of H, F, and Cl; and/or Y¹, Y² are independently of each other selected from the group consisting of O and S.

According to the present invention it is also preferred that in the compound of formula (I) A and B are independently of each other selected from the group consisting of phenyl, substituted phenyl, naphthyl, substituted naphthyl, biphenyl, substituted biphenyl, terphenyl, substituted terphenyl, anthranyl, substituted anthranyl, pyrenyl, substituted pyrenyl, perylenyl, substituted perylenyl, thienyl, substituted thienyl, pyridyl, substituted pyridyl, quinolinyl, substituted quinolinyl, bithienyl, substituted bithienyl, terthienyl, substituted terthienly, wherein any substituent, if present, is a substituent Z², where each Z² is independently of each other selected from the group consisting of alkyl having 1 to 12 carbon atoms, O(alkyl) having 1 to 6 carbon atoms, F, Cl, CN, dicyanovinyl, cyanopyridylvinyl, cyanophenylvinyl, phenyl, naphthyl, thienyl, bithienyl, and pyridyl.

According to the present invention, in the compound of formula (I) A or B or each of A and B independently of each other indicates a 5- or 6-membered ring present in a moiety selected from the group consisting of 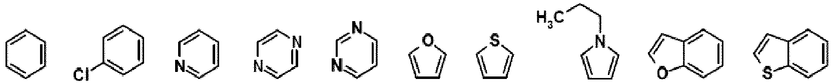 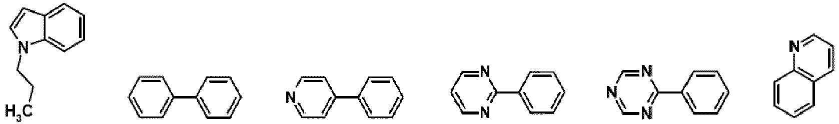 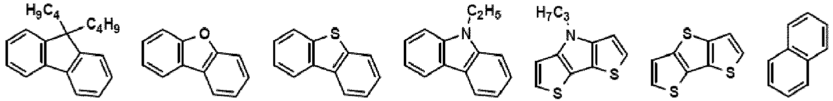 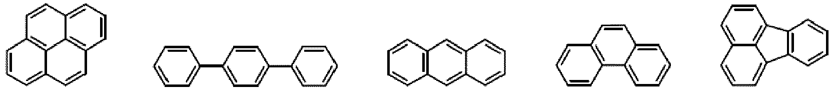 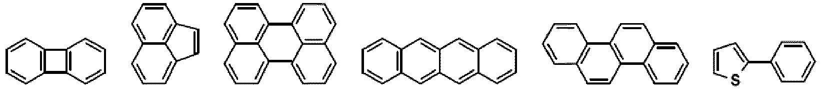 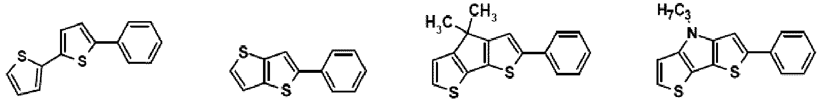 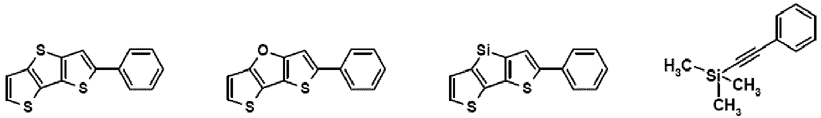 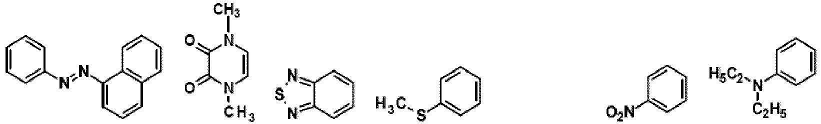 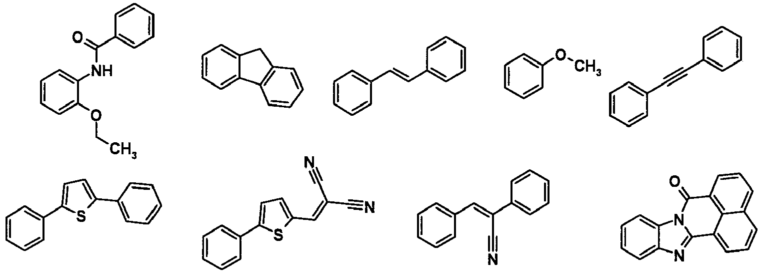 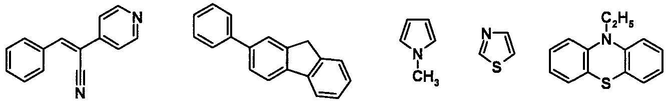 wherein in each 5- or 6-membered ring present in said moieties and independently of each other indicating A or B, respectively, two adjacent carbon atoms are identical with carbon atoms marked with * in formula (I).

This situation is explained in detail using the example of the 2-phenylthiophene moiety. As shown below for the 2-phenylthiophene moiety either the phenyl ring or the thiophene ring may indicate the 5- or 6-membered ring A or B, respectively. Further, for each 5- or 6-membered ring A or B, respectively, there exist two possible ways for two adjacent carbon atoms to be identical with carbon atoms marked with * in formula (I). Thus, the 2-phenylthiophene moiety shown above is meant to cover all of the following structural elements:

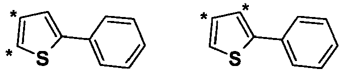

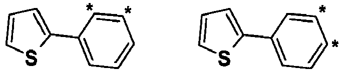

In a similar manner, all other moieties shown above are meant to cover all structural elements that can be realized.

Exemplary compounds of formula (I) are the following:

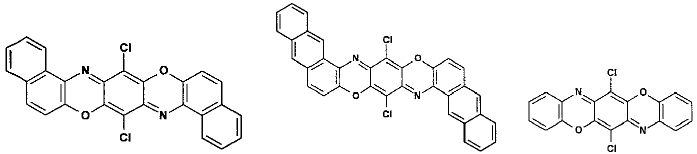

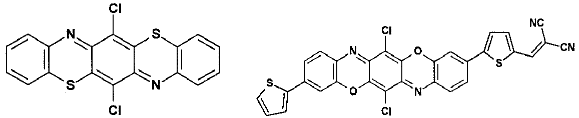

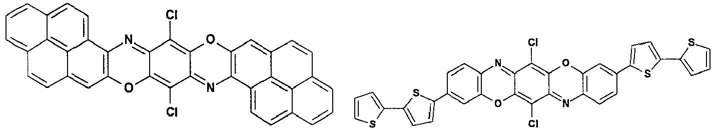

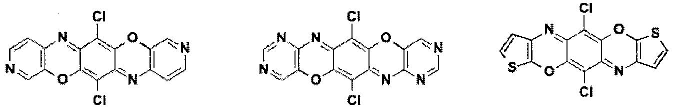

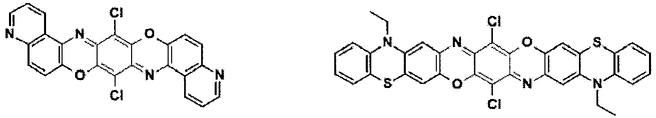

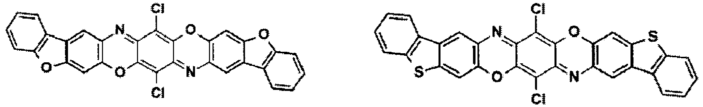

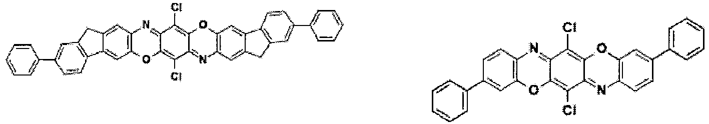

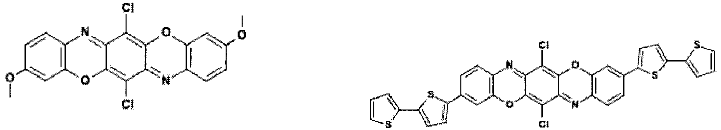

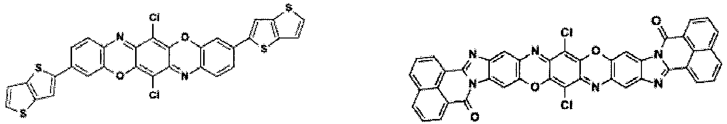

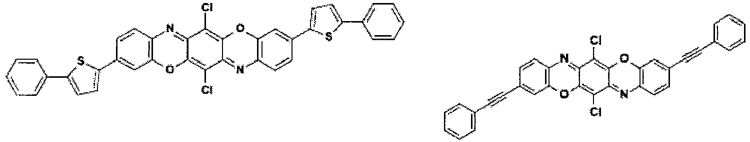

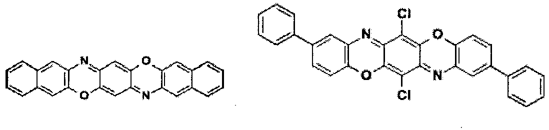

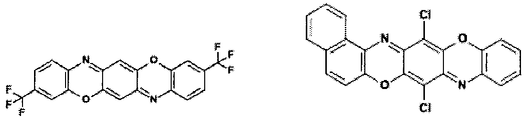

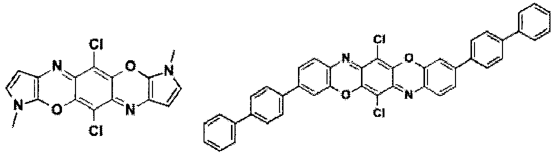

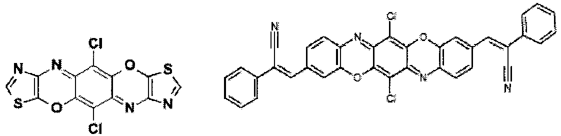

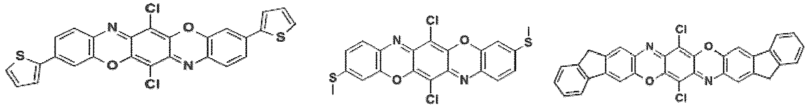

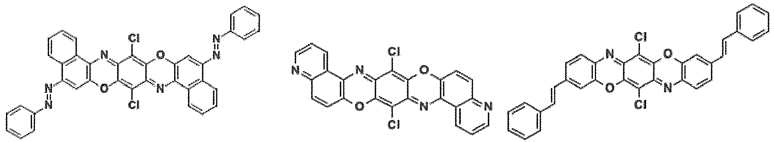

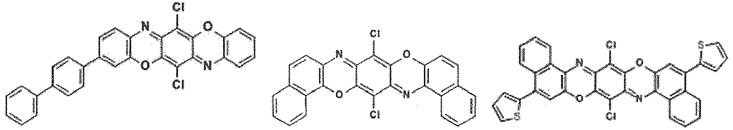

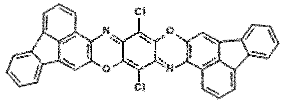

The compounds of formula (I) as defined above are especially suitable for use as donor substance or as acceptor substance in a photoactive material, wherein the photoactive material is part of a layer of a donor-acceptor bilayer or part of a donor-acceptor mixed layer (bulk heterojunction, BHJ).

According to the present invention it is preferred that the inventive photoactive material as described above further comprises one or more semiconductor material(s).

According to the present invention it is preferred that the photoactive material as described above comprises at least one compound of formula (I) as donor substance, which is in contact with at least one fullerene or fullerene derivative as acceptor substance, such as C₆₀, C₇₀, C₈₄, phenyl-C₆₁-butyric acid methyl ester ([60]PCBM), phenyl-C₇₁-butyric acid methyl ester ([70]PCBM), phenyl-C₈₅-butyric acid methyl ester ([84]PCBM), phenyl-C₆₁-butyric acid butyl ester ([60]PCBB), phenyl-C₆₁-butyric acid octyl ester ([60]PCBO), thienyl-C₆₁-butyric acid methyl ester ([60]ThCBM), [6,6]-phenyl-C₆₁-butyric acid methyl ester and mixtures thereof. Particular preference is given to C₆₀, C₇₀, [6,6]-phenyl-C₆₁-butyric acid methyl ester and mixtures thereof. Preference is also given to 3,4,9,10-perylenetetracarboxyl-bisbenzimidazole (PTCBI) as acceptor substance.

According to the present invention it is especially preferred that the photoactive material as described above comprises a donor substance and an acceptor substance, wherein the donor substance comprises or consists of one or more compounds of formula (I) described above, and wherein the acceptor substance comprises or consists of one or more compounds selected from the group of
(i) fullerenes and fullerene derivatives, preferably selected from the group consisting of C₆₀, C₇₀ and [6,6]-phenyl-C₆₁-butyric acid methyl ester, and
(ii) 3,4,9,10-perylenetetracarboxyl-bisbenzimidazole (PTCBI).

The invention also provides an organic solar cell comprising a photoactive material as defined above.

Organic solar cells generally have a layer structure and generally comprise at least the following layers: anode, photoactive layer and cathode. These layers are generally applied to a substrate suitable for this purpose. The structure of organic solar cells is described, for example, in US 2005/0098726 and US 2005/0224905.

The inventive organic solar cell comprises at least one photoactive material. The photoactive material comprises a donor substance and an acceptor substance, wherein preferably the donor substance and the acceptor substance are part of respective layers of a donor-acceptor bilayer or part of a donor-acceptor mixed layer (bulk heterojunction, BHJ).

Organic solar cells with photoactive donor-acceptor transitions in the form of a bulk heterojunction are especially preferred according to the present invention.

Suitable substrates for organic solar cells are, for example, oxidic materials, polymers and combinations thereof. Preferred oxidic materials are selected from glass, ceramic, SiO₂, quartz, etc. Preferred polymers are selected from polyethylene terephthalates, polyolefins (such as polyethylene and polypropylene), polyesters, fluoropolymers, polyamides, polyurethanes, polyalkyl (meth)acrylates, polystyrenes, polyvinyl chlorides and mixtures and composites.

Suitable electrodes (cathode, anode) are in principle semiconductors, metal alloys, semiconductor alloys and combinations thereof. Preferred metals are those of groups 2, 8, 9, 10, 11 or 13 of the periodic table, e.g. Pt, Au, Ag, Cu, Al, In, Mg or Ca.

Preferred semiconductors are, for example, doped Si, doped Ge, indium tin oxide (ITO), fluorinated tin oxide (FTO), gallium indium tin oxide (GITO), zinc indium tin oxide (ZITO), etc. Preferred metal alloys are for example alloys based on Pt, Au, Ag, Cu, etc. Especially preferred are Mg/Ag alloys.

The material used for the electrode facing the light (the anode in a normal structure, the cathode in an inverse structure) is preferably a material at least partly transparent to the incident light. This preferably includes electrodes which have glass and/or a transparent polymer as a carrier material. Transparent polymers suitable as carriers are those mentioned above, such as polyethylene terephthalate. The electrical contact connection is generally effected by means of metal layers and/or transparent conductive oxides (TCOs). These preferably include ITO, doped ITO, FTO (fluorine doped tin oxide), AZO (aluminum doped tin oxide), ZnO, TiO₂, Ag, Au, Pt. Particular preference is given to ITO for contact connection. For electrical contact connection, it is also possible to use a conductive polymer, for example a poly-3,4-alkylenedioxy-thiophene, e.g. poly-3,4-ethyleneoxythiophene (PEDOT).

The electrode facing the light is configured such that it is sufficiently thin to bring about only minimal light absorption but thick enough to enable good charge transport of the extracted charge carriers. The thickness of the electrode layer (without carrier material) is preferably within a range from 20 to 200 nm.

It is preferred that the material used for the electrode facing away from the light (the cathode in a normal structure, the anode in an inverse structure) is a material which at least partly reflects the incident light. This includes metal films, preferably of Ag, Au, Al, Ca, Mg, In, and mixtures thereof. Preferred mixtures are Mg/Al. The thickness of the electrode layer is preferably within a range from 50 to 300 nm.

The photoactive region comprises or consists of at least one layer comprising or consisting of a photoactive material as defined above. In addition, the photoactive region may have one or more further layer(s). These are, for example, selected from
layers with electron-conducting properties (electron transport layer, ETL),
layers which comprise a hole-conducting substance (hole transport layer, HTL), which need not absorb any radiation,
exciton- and hole-blocking layers (e.g. EBLs), which must not absorb, and
multiplication layers.

Suitable materials for these layers are described in detail hereinafter. Suitable exciton- and hole-blocking layers are described, for example, in US 6,451,415. Suitable materials for exciton-blocking layers are, for example, bathocuproine (BCP), 4,4',4"-tris[3-methylphenyl-N-phenylamino]triphenylamine (m-MTDATA) or polyethylenedioxythiophene (PEDOT).

The inventive organic solar cells comprise at least one photoactive donor-acceptor heterojunction. Optical excitation of an organic substance generates excitons. In order that a photocurrent occurs, the electron-hole pair has to be separated, typically at a donor-acceptor interface between two unlike contact materials. At such an interface, the donor substance forms a heterojunction with an acceptor substance. When the charges are not separated, they can recombine in a process also known as "quenching", either radiatively by the emission of light of a lower energy than the incident light or nonradiatively by generation of heat. Both processes are undesired. According to the invention, at least one compound of formula (I) can be used as a charge generator (donor). In combination with an appropriate electron acceptor substance (ETM, electron transport material), radiative excitation is followed by a rapid electron transfer to the ETM. Suitable ETMs are C₆₀ and other fullerenes.

It is preferred that the heterojunction has a flat configuration, i.e. a bilayer configuration (see: Two layer organic photovoltaic cell, C. W. Tang, Appl. Phys. Lett. 1986, 48 (2), 183-185 or N. Karl, A. Bauer, J. Holzapfel, J. Marktanner, M. Mobus, F. Stolzle, Mol. Cryst. Liq.Cryst. 1994, 252, 243-258).

It is also preferred that the heterojunction is configured as a bulk (mixed) heterojunction, also referred to as an interpenetrating donor-acceptor network. Organic photovoltaic cells with a bulk heterojunction are described, for example, by C. J. Brabec, N. S. Sariciftci, J. C. Hummelen in Adv. Funct. Mater. 2001, 11 (1), 15 or by J. Xue, B. P. Rand, S. Uchida and S. R. Forrest in J. Appl. Phys. 2005, 98, 124903. Bulk heterojunctions are discussed in detail hereinafter.

The compounds of the formula (I) can be used in a photoactive material in cells with MiM, pin, pn, Mip or Min structure (M = metal, p = p-doped organic or inorganic semiconductor, n = n-doped organic or inorganic semiconductor, i = intrinsically conductive system of organic layers; see, for example, J. Drechsel et al., Org. Electron. 2004, 5 (4), 175 or Maennig et al., Appl. Phys. A 2004, 79, 1-14).

The compounds of the formula (I) can also be used in a photoactive material in tandem cells. Suitable tandem cells are described, for example, by P. Peumans, A. Yakimov, S. R. Forrest in J. Appl. Phys. 2003, 93 (7), 3693-3723 (see also US 4,461, 922, US 6,198,091 and US 6,198,092) and are described in detail hereinafter. The use of compounds of the general formula (I) in tandem cells is preferred according to the present invention.

The compounds of the formula (I) can also be used in a photoactive material in tandem cells which are constructed from two or more than two stacked MiM, pin, Mip or Min structures (see DE 103 13 232.5 and J. Drechsel et al., Thin Solid Films 2004, 451-452, 515-517).

The layer thickness of M, n, i and p layers is typically within a range from 10 to 1000 nm, more preferably from 10 to 400 nm. The layers which form the solar cell can be produced by customary processes known to those skilled in the art. These include vapor deposition under reduced pressure or in an inert gas atmosphere, laser ablation or solution or dispersion processing methods such as spincoating, knifecoating, casting methods, spray application, dipcoating or printing (e.g. inkjet, flexographic, offset, gravure; intaglio, nanoimprinting). It is preferred that the entire solar cell is produced by a gas phase deposition process.

In order to improve the efficiency of organic solar cells, it is possible to shorten the mean distance through which the exciton has to diffuse in order to arrive at the next donor-acceptor interface. To this end, it is possible to use mixed layers of donor substances and acceptor substances which form an interpenetrating network in which internal donor-acceptor heterojunctions are possible. This bulk heterojunction is a specific form of the mixed layer, in which the excitons generated need only travel a very short distance before they arrive at a domain boundary, where they are separated.

It is preferred that the photoactive donor-acceptor transitions in the form of a bulk heterojunction are produced by a gas phase deposition process (physical vapor deposition, PVD). Suitable processes are described, for example, in US 2005/0227406, to which reference is made here. To this end, a compound of the general formula (I) and a complementary semiconductor material can be subjected to a gas phase deposition in the manner of a cosublimation. PVD processes are performed under high-vacuum conditions and comprise the following steps: evaporation, transport, deposition. The deposition is effected preferably at a pressure within a range from about 10⁻² mbar to 10⁻⁷ mbar, for example from 10⁻⁵ to 10⁻⁷ mbar. The deposition rate is preferably within a range from 0.01 to 10 nm/s. The deposition can be effected in an inert gas atmosphere, for example under nitrogen, helium or argon. The temperature of the substrate during the deposition is preferably within a range from -100 to 300°C, more preferably from -50 to 250°C.

The other layers of the organic solar cell can be produced by known processes. These include vapor deposition under reduced pressure or in an inert gas atmosphere, laser ablation, or solution or dispersion processing methods such as spincoating, knifecoating, casting methods, spray application, dipcoating or printing (e.g. inkjet, flexographic, offset, gravure; intaglio, nanoimprinting). It is especially preferred that the entire solar cell is produced by a gas phase deposition process.

The photoactive layer (homogeneous layer or mixed layer) can be subjected to a thermal treatment directly after production thereof or after production of further layers which form the solar cell. Such a heat treatment can in many cases further improve the morphology of the photoactive layer. The temperature is preferably within a range from about 60°C to 300°C. The treatment time is preferably within a range from 1 minute to 3 hours. In addition or alternatively to a thermal treatment, the photoactive layer (mixed layer) can be subjected to a treatment with a solvent-containing gas directly after production thereof or after production of further layers which form the solar cell. In this respect, saturated solvent vapors in air are used at ambient temperature. Suitable solvents are toluene, xylene, chloroform, N-methylpyrrolidone, dimethylformamide, ethyl acetate, chlorobenzene, dichloromethane and mixtures thereof. The treatment time is preferably within a range from 1 minute to 3 hours.

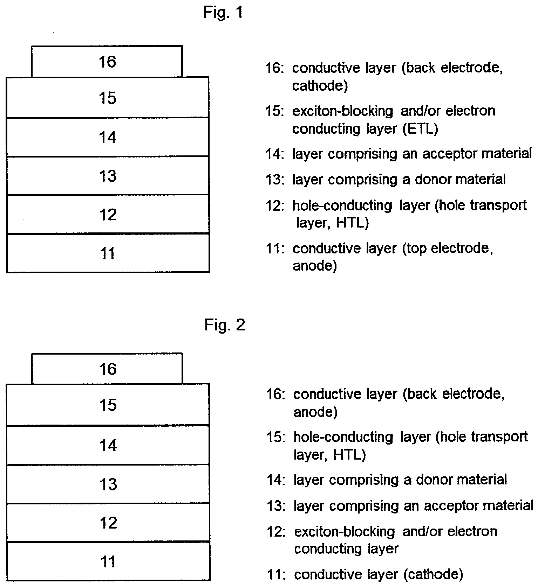

It is preferred that the inventive organic solar cells are present as an individual cell with flat heterojunction normal structure. Figure 1 shows an inventive solar cell with normal structure. It is especially preferred that the cell has the following structure:
an at least partly transparent conductive layer (top electrode, anode) (11)
a hole-conducting layer (hole transport layer, HTL) (12)
a layer which comprises a donor substance (13)
a layer which comprises an acceptor substance (14)
an exciton-blocking and/or electron-conducting layer (15)
a second conductive layer (back electrode, cathode) (16)

The donor substance preferably comprises at least one compound of formula (I) or consists of a compound of the formula (I). According to the present invention it is preferred that the acceptor substance comprises at least one fullerene or fullerene derivative, or consists of a fullerene or fullerene derivative. The acceptor substance preferably comprises C₆₀, C₇₀, [6,6]-phenyl-C61-butyric acid methyl ester or PTCBI (3,4,9,10-perylenetetracarboxyl-bisbenzimidazole).

The essentially transparent conductive layer (11) (anode) comprises a carrier, such as glass or a polymer (e.g. polyethylene terephthalate) and a conductive material, as described above. Examples include ITO, doped ITO, FTO, ZnO, AZO, etc. The anode material can be subjected to a surface treatment, for example with UV light, ozone, oxygen plasma, Br₂, etc. The layer (11) should be sufficiently thin to enable maximum light absorption, but also sufficiently thick to ensure good charge transport. The layer thickness of the transparent conductive layer (11) is preferably within a range from 20 to 200 nm.

The solar cell with normal structure according to figure 1 optionally has a hole-conducting layer (HTL). This layer comprises at least one hole-conducting substance (hole transport material, HTM). Layer (12) may be an individual layer of essentially homogeneous composition or may comprise two or more than two sublayers.

Hole-conducting materials (HTM) suitable for forming layers with hole-conducting properties (HTL) preferably comprise at least one substance with high ionization energy. The ionization energy is preferably at least 5.0 eV, more preferably at least 5.5 eV. The substance may be organic or inorganic. Organic substances suitable for use in a layer with hole-conducting properties are preferably selected from poly(3,4-ethylenedioxythiophene) poly(styrenesulfonate) (PEDOT-PSS), Ir-DPBIC (tris-N,N-diphenylbenzimidazol-2-ylideneiridium(III), N,N'-diphenyl-N,N'-bis(3-methylphenyl)-1,1'-diphenyl-4,4'-diamine (a-NPD), 2,2',7,7'-tetrakis(N,N-di-p-methoxyphenylamine)-9,9'-spirobifluorene (spiro-MeOTAD), etc. and mixtures thereof. The organic substances may, if desired, be doped with a p-dopant which has a LUMO within the same range as or lower than the HOMO of the hole-conducting substance. Suitable dopants are, for example, 2,3,5,6-tetrafluoro-7,7,8,8-tetracyanoquinodimethane (F4TCNQ), WO₃, MoO₃, etc. Inorganic substances suitable for use in a layer with hole-conducting properties are preferably selected from WO₃, MoO₃, etc.

If present, the thickness of the layers with hole-conducting properties is preferably within a range from 5 to 200 nm, more preferably 10 to 100 nm.

Layer (13) comprises at least one compound of general formula (I). The thickness of the layer should be sufficient to absorb a maximum amount of light, but thin enough to enable effective dissipation of the charge. The thickness of layer (13) is preferably within a range from 5 nm to 1 µm, more preferably from 5 to 80 nm.

Layer (14) comprises at least one acceptor substance. According to the present invention it is preferred that the acceptor substance comprises at least one fullerene or fullerene derivative. The thickness of the layer should be sufficient to absorb a maximum amount of light, but thin enough to enable effective dissipation of the charge. The thickness of layer (14) is preferably within a range from 5 nm to 1 µm, more preferably from 5 to 80 nm.

The solar cell with normal structure according to figure 1 optionally comprises an exciton-blocking and/or electron-conducting layer (15) (EBL/ETL). Suitable substances for exciton-blocking layers generally have a greater band gap than the substances of layer (13) and/or (14). They are firstly capable of reflecting excitons and secondly enable good electron transport through the layer. The materials for the layer (15) may comprise organic or inorganic substances. Suitable organic substances are preferably selected from 2,9-dimethyl-4,7-diphenyl-1,10-phenanthroline (BCP), 4,7-diphenyl-1,10-phenanthroline (Bphen), 1,3-bis[2-(2,2'-bipyridin-6-yl)-1,3,4-oxadiazo-5-yl]benzene (BPY-OXD), etc. The organic substances may, if desired, be doped with an n-dopant which has a HOMO within the same range as or lower than the LUMO of the electron-conducting substance. Suitable dopants are, for example, Cs₂CO₃, Pyronin B (PyB), Rhodamine B, cobaltocenes, etc. Inorganic materials suitable for use in a layer with electron-conducting properties are preferably selected from ZnO, etc. If present, the thickness of the layer (15) is preferably within a range from 5 to 500 nm, more preferably 10 to 100 nm.

Layer (16) is the cathode and preferably comprises at least one compound with low work function, more preferably a metal such as Ag, Al, Mg, Ca, etc. The thickness of the layer (16) is preferably within a range from about 10 nm to 10 µm, e.g. 10 nm to 60 nm.

It is also preferred that the inventive solar cells are present as an individual cell with a flat heterojunction and inverse structure. Figure 2 shows a solar cell with inverse structure. It is especially preferred that the cell has the following structure:
an at least partly transparent conductive layer (cathode) (11)
an exciton-blocking and/or electron-conducting layer (12)
a layer which comprises an acceptor substance (13)
a layer which comprises a donor substance (14)
a hole-conducting layer (hole transport layer, HTL) (15)
a second conductive layer (back electrode, anode) (16)

With regard to suitable and preferred materials for the layers (11) to (16), reference is made to the above remarks regarding the corresponding layers in solar cells with normal structure.

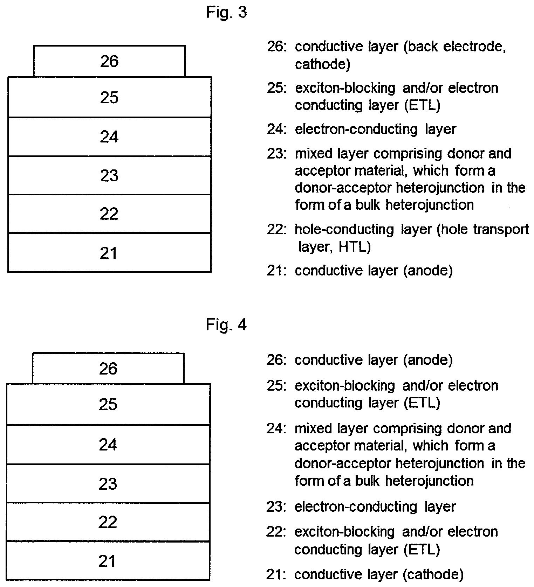

According to the present invention it is also preferred that the inventive organic solar cells are present as an individual cell with normal structure and have a bulk heterojunction. Figure 3 shows a solar cell with a bulk heterojunction. It is especially preferred that the cell has the following structure:
an at least partly transparent conductive layer (anode) (21)
a hole-conducting layer (hole transport layer, HTL) (22)
a mixed layer which comprises a donor substance and an acceptor substance, which form a donor-acceptor heterojunction in the form of a bulk heterojunction (23)
an electron-conducting layer (24)
an exciton-blocking and/or electron-conducting layer (25)
a second conductive layer (back electrode, cathode) (26)

The layer (23) comprises at least one a photoactive material comprising at least one compound of general formula (I), especially as a donor substance. The layer (23) additionally comprises preferably at least one fullerene or fullerene derivative as an acceptor substance. The layer (23) preferably comprises C₆₀ or PTCBI (3,4,9,10-perylenetetracarboxyl-bisbenzimidazole) as an acceptor substance. With regard to layer (21), reference is made completely to the above remarks regarding layer (11).

With regard to layer (22), reference is made completely to the above remarks regarding layer (12).

Layer (23) is a mixed layer which comprises at least one compound of the general formula (I) as a donor substance. In addition, layer (23) comprises at least one acceptor substance. As described above, layer (23) can be produced by coevaporation or by solution processing using customary solvents. The mixed layer comprises preferably 10 to 90% by weight, more preferably 20 to 80% by weight, of at least one compound of the general formula (I), based on the total weight of the mixed layer. The mixed layer comprises preferably 10 to 90% by weight, more preferably 20 to 80% by weight, of at least one acceptor substance, based on the total weight of the mixed layer. The thickness of the layer (23) should be sufficient to absorb a maximum amount of light, but thin enough to enable effective dissipation of the charge. The thickness of the layer (23) is preferably within a range from 5 nm to 1 µm, more preferably from 5 to 200 nm, most preferably from 5 to 80 nm.

The solar cell with a bulk heterojunction according to figure 3 comprises an electron-conducting layer (24) (ETL). This layer comprises at least one electron transport material (ETM). Layer (24) may be a single layer of essentially homogeneous composition or may comprise two or more than two sublayers. Suitable materials for electron-conducting layers generally have a low work function or ionization energy. The ionization energy is preferably not more than 3.5 eV. Suitable organic substances are preferably selected from the aforementioned fullerenes and fullerene derivatives, 2,9-dimethyl-4,7-diphenyl-1,10-phenanthroline (BCP), 4,7-diphenyl-1,10-phenanthroline (Bphen), 1,3-bis[2-(2,2'-bipyridin-6-yl)-1,3,4-oxadiazo-5-yl]benzene (BPY-OXD), etc. The organic substances used in layer (24) may, if desired, be doped with an n-dopant which has a HOMO within the same range as or lower than the LUMO of the electron-conducting substance. Suitable dopants are, for example, CS₂CO₃, Pyronin B (PyB), Rhodamine B, cobaltocenes, etc. The thickness of the layer (23) is, if present, preferably within a range from 1 nm to 1 µm, more preferably from 5 to 60 nm.

With regard to layer (25), reference is made completely to the above remarks regarding layer (15).

With regard to layer (26), reference is made completely to the above remarks regarding layer (16).

The solar cell with a donor-acceptor heterojunction in the form of a bulk heterojunction can be produced by a gas phase deposition process as described above. With regard to deposition rates, substrate temperature during the deposition and thermal aftertreatment, reference is made to the above remarks.

According to the present invention it is also preferred that the inventive organic solar cells are present as an individual cell with inverse structure and have a bulk heterojunction. Figure 4 shows a solar cell with a bulk heterojunction and inverse structure.

The present invention also provides a photoelectric conversion device comprising or consisting of two or more organic solar cells as described above.

A photoelectric conversion device consists of two or more than two (e.g. 3, 4, 5) solar cells. A single solar cell, some of the solar cells or all solar cells may have photoactive donor-acceptor heterojunctions. Each donor-acceptor-heterojunction may be in the form of a flat heterojunction or in the form of a bulk heterojunction. Preferably, at least one of the donor-acceptor heterojunctions is in the form of a bulk heterojunction. According to the invention, the photoactive material of at least one solar cell comprises a compound of general formula (I). Preferably, the photoactive material of at least one solar cell comprises a compound of general formula (I) and at least one fullerene or fullerene derivative. More preferably, the semiconductor mixture used in the photoactive layer of at least one solar cell consists of a compound of formula (I) and C₆₀, C₇₀ or [6,6]-phenyl-C₆₁-butyric acid methyl ester.

The solar cells which are arranged as tandem cells may be connected in parallel or in series. The solar cells which are arranged as tandem cells are preferably connected in series. There is preferably an additional recombination layer in each case between the individual solar cells. The individual solar cells have the same polarity, i.e. generally either only cells with normal structure or only cells with inverse structure are combined with one another.

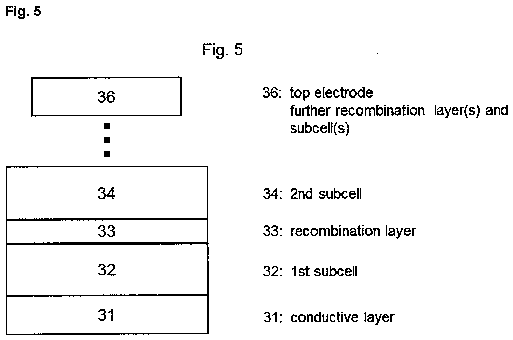

Figure 5 shows the basic structure of a photoelectric conversion device in which the solar cells are arranged as tandem cells. Layer 31 is a transparent conductive layer. Suitable materials are those specified above for the individual cells.

Layers (32) and (34) constitute subcells. "Subcell" refers here to a cell as defined above without cathode and anode. The subcells may, for example, either all have a compound of general formula (I) used in accordance with the invention in the photoactive material (preferably in combination with a fullerene or fullerene derivative, especially C₆₀) or have other combinations of semiconductor materials, for example C₆₀ with zinc phthalocyanine, C₆₀ with oligothiophene (such as DCV5T). In addition, individual subcells may also be configured as dye-sensitized solar cells or polymer cells. In all cases, preference is given to a combination of materials which exploit different regions of the spectrum of the incident light, for example of natural sunlight. For instance, the combination of a compound of general formula (I) and a fullerene or a fullerene derivative used in accordance with the invention absorbs in the long-wave region of sunlight. Dibenzoperiflanthene(DBP)-C₆₀ absorbs primarily in the range from 400 nm to 600 nm. Zinc phthalocyanine-C₆₀ cells absorb primarily in the range from 600 nm to 800 nm. Thus, a photoelectric conversion device composed of a combination of these subcells should absorb radiation in the range from 400 nm to 800 nm. Suitable combination of subcells should thus allow the spectral range utilized to be extended. For optimal performance properties, optical interference should be considered. For instance, subcells which absorb at relatively short wavelengths should be arranged closer to the metal top contact than subcells with longer-wave absorption.

With regard to layer (31), reference is made completely to the above remarks regarding layers (11) and (21).

With regard to layers (32) and (34), reference is made completely to the above remarks regarding layers (12) to (15) for flat heterojunctions and (22) to (25) for bulk heterojunctions.

Layer (33) is a recombination layer. Recombination layers enable the charge carriers from one subcell to recombine with those of an adjacent subcell. Small metal clusters are suitable, such as Ag, Au or combinations of highly n- and p-doped layers. In the case of metal clusters, the layer thickness is preferably within a range from 0.5 to 5 nm. In the case of highly n- and p-doped layers, the layer thickness is preferably within a range from 5 to 40 nm. The recombination layer generally connects the electron-conducting layer of a subcell to the hole-conducting layer of an adjacent subcell. In this way, further cells can be combined to form the photoelectric conversion device.

Layer (36) is the top electrode. The material depends on the polarity of the subcells. For subcells with normal structure, preference is given to using metals with a low work function, such as Ag, Al, Mg, Ca, etc. For subcells with inverse structure, preference is given to using metals with a high work function, such as Au or Pt, or PEDOT-PSS.

In the case of subcells connected in series, the overall voltage corresponds to the sum of the individual voltages of all subcells. The overall current, in contrast, is limited by the lowest current of one subcell. For this reason, the thickness of each subcell should be optimized such that all subcells have essentially the same current.

Examples of different kinds of donor-acceptor heterojunctions are a donor-acceptor double layer with a flat heterojunction, or the heterojunction is configured as a hybrid planar-mixed heterojunction or gradient bulk heterojunction or annealed bulk heterojunction.

The production of a hybrid planar-mixed heterojunction is described in Adv. Mater. 2005, 17, 66-70. In this structure, mixed heterojunction layers, which were formed by simultaneous evaporation of acceptor and donor material, are present between homogeneous donor and acceptor material.

According to the present invention it is preferred that the organic solar cells of the inventive photoelectric conversion device are arranged as tandem cells (multi-junction solar cells) or as inverted tandem cells.

In addition to fullerenes, the semiconductor materials listed hereinafter are suitable in principle for use in the inventive organic solar cells. They serve as donors or acceptors for subcells of a tandem cell, which are combined with a subcell according to the present invention.

Suitable further semiconductors are phthalocyanines. These include phthalocyanines which are nonhalogenated or which bear 1 to 16 halogen atoms. The phthalocyanines may be metal-free or contain a divalent metal or a metal atom-containing group.

Preference is given to phthalocyanines based on zinc, copper, iron, titanyloxy, vanadyloxy, etc. Particular preference is given to copper phthalocyanines, zinc phthalocyanines, metal-free phthalocyanines. It is preferred that halogenated phthalocyanines are used. These include:
2,6,10,14-tetrafluorophthalocyanines, e.g. copper 2,6,10,14-tetrafluorophthalocyanine and zinc 2,6,10,14-tetrafluorophthalocyanine;
1,5,9,13-tetrafluorophthalocyanines, e.g. copper 1,5,9,13-tetrafluorophthalocyanines and zinc 1,5,9,13-tetrafluorophthalocyanines;
2,3,6,7,10,11,14,15-octafluorophthalocyanine, e.g. copper 2,3,6,7,10,11,14,15-octafluorophthalocyanine and zinc 2,3,6,7,10,11,14,15-octafluorophthalocyanine;
phthalocyanines which are suitable as acceptors are, for example, hexadecachlorophthalocyanines and hexadecafluorophthalocyanines, such as copper hexadecachlorophthalocyanine, zinc hexadecachlorophthalocyanine, metal-free hexadecachlorophthalocyanine, copper hexadecafluorophthalocyanine, hexadecafluorophthalocyanine or metal-free hexadecafluorophthalocyanine.

Preferred features of the present invention are explained in the claims and the specification. It is understood that combinations of preferred features are within the scope of the present invention.

The invention is hereinafter further illustrated by means of the following examples.

### Preparation Examples of compounds of formula (I)

### Compound 1

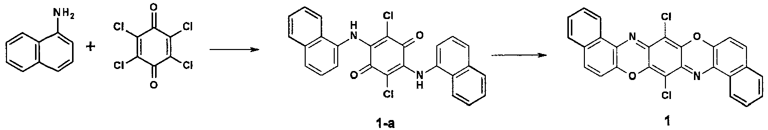

### Preparation of intermediate 1-a

In a 300 ml reactor 2.45 g (10 mmol) of chloranil, 4.1 g (50 mmol) of AcONa and 75 ml of ethanol are placed under N₂ atmosphere. The solution of 2.9 g (20 mmol) of 1-aminonaphthalene in 75 ml of ethanol is slowly added to the reactor. After stirring at room temperature for 30 minutes, the mixture is heated up to reflux temperature. After the reaction is terminated, the mixture is filtrated hot and the solid is washed with hot H₂O, ethanol and tert-butylmethylether (TBME). 1.9 g of intermediate **1-a** is obtained after drying under reduced pressure.
Yield: 41 %
Appearance: dark brown solid
¹H-NMR (DMSO): 9.8 ppm (2H, s), 6.8-7.5 ppm (12H, m)
Melting Point: 340°C

### Preparation of compound 1

In a 50 ml reactor 1.5 g (3.27 mmol) of intermediate **1-a,** 4.5 ml of benzoylchloride and 15 ml of nitrobenzene are placed under N₂ atmosphere. The mixture is stirred at 215°C for 7 hours. The reaction mixture is filtered hot, and then the solid is washed with ethanol, H₂O and dichloromethane. 0.51 g of compound **1** is obtained after drying under reduced pressure.
Yield: 35 %
Appearance: green metallic crystals
Melting Point: 427°C
UV-VIS (DMF): 540, 580 nm

**The following compounds can be prepared in analogy to compound 1:**

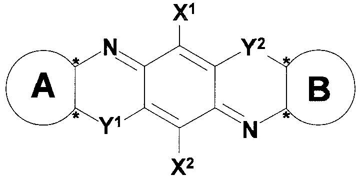

| Compound | X¹, X² | Y¹, Y² | moiety comprising A | moiety comprising B |
|---|---|---|---|---|
| 2 | Cl | O | 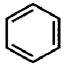 | 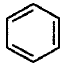 |
| 3 | Cl | O | 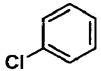 | 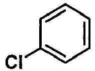 |
| 4 | Cl | O | 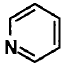 | 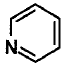 |
| 5 | Cl | O | 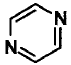 | 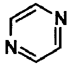 |
| 6 | Cl | O | 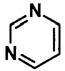 | 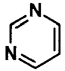 |
| 7 | Cl | O | 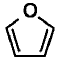 | 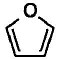 |
| 8 | Cl | O | 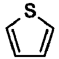 | 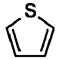 |
| 9 | Cl | O | 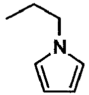 | 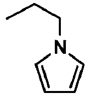 |
| 10 | Cl | O | 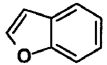 | 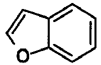 |
| 11 | Cl | O | 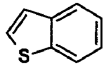 | 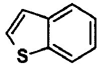 |
| 12 | Cl | O | 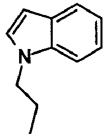 | 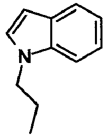 |
| 13 | Cl | O | 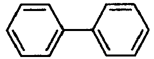 | 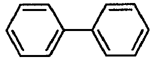 |
| 14 | Cl | O | 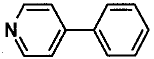 | 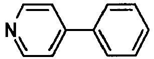 |
| 15 | Cl | O | 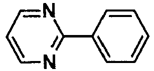 | 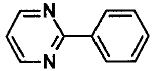 |
| 16 | Cl | O | 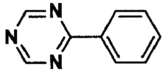 | 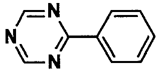 |
| 17 | Cl | O | 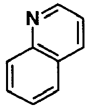 | 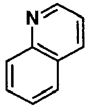 |
| 18 | Cl | O | 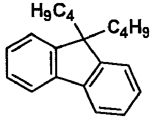 | 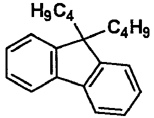 |
| 19 | Cl | O | 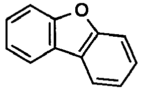 | 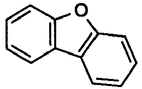 |
| 20 | Cl | O | 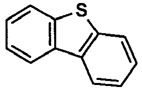 | 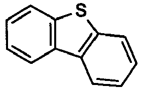 |
| 21 | Cl | O | 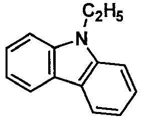 | 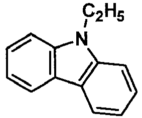 |
| 22 | Cl | O | 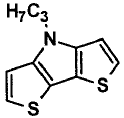 | 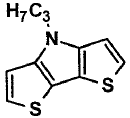 |
| 23 | Cl | O | 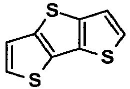 | 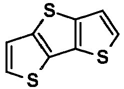 |
| 24 | Cl | O | 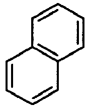 | 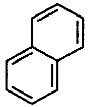 |
| 25 | Cl | O | 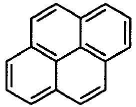 | 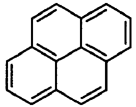 |
| 26 | Cl | O | 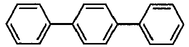 | 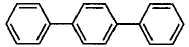 |
| 27 | Cl | O | 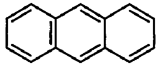 | 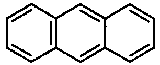 |
| 28 | Cl | O | 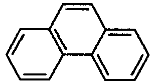 | 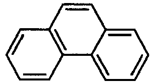 |
| 29 | Cl | O | 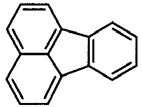 | 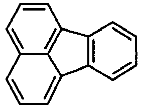 |
| 30 | Cl | O | 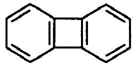 | 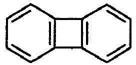 |
| 31 | Cl | O | 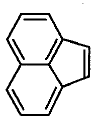 | 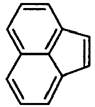 |
| 32 | Cl | O | 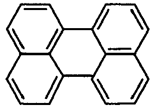 | 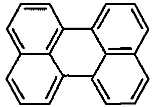 |
| 33 | Cl | O | 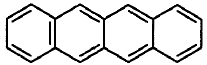 | 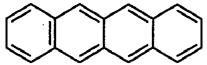 |
| 34 | Cl | O | 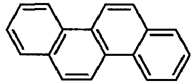 | 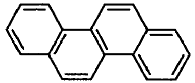 |
| 35 | Cl | O | 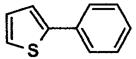 | 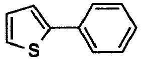 |
| 36 | Cl | O | 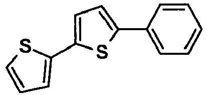 | 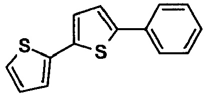 |
| 37 | Cl | O | 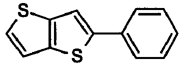 | 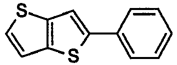 |
| 38 | Cl | O | 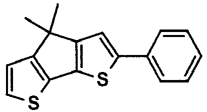 | 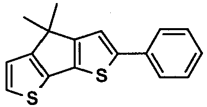 |
| 39 | Cl | O | 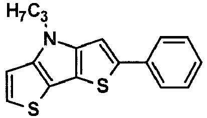 | 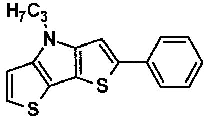 |
| 40 | Cl | O | 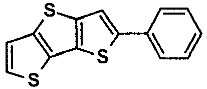 | 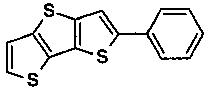 |
| 41 | Cl | O | 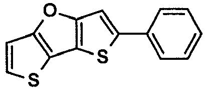 | 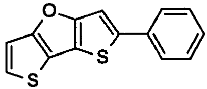 |
| 42 | Cl | O | 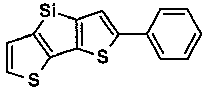 | 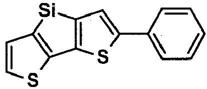 |
| 43 | Cl | O | 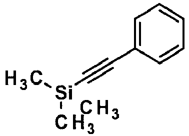 | 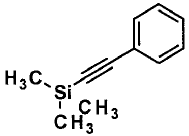 |
| 44 | Cl | O | 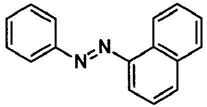 | 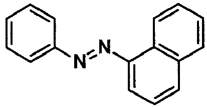 |
| 45 | Cl | O | 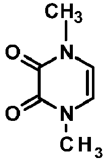 | 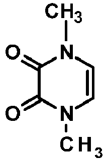 |
| 46 | Cl | O | 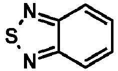 | 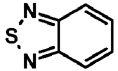 |
| 47 | Cl | S | 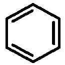 | 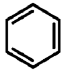 |
| 48 | Cl | S | 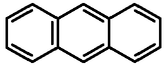 | 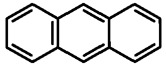 |
| 49 | Cl | S | 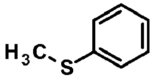 | 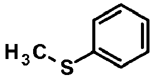 |
| 50 | F | S | 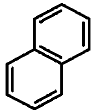 | 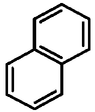 |
| 51 | F | O | 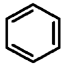 | 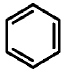 |
| 52 (not according to the invention) | H | O | 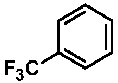 | 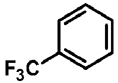 |
| 53 | H | O | 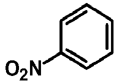 | 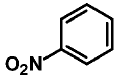 |
| 54 | CN | O | 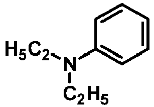 | 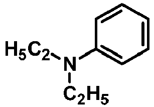 |
| 55 | CN | S | 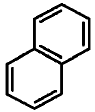 | 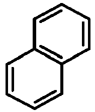 |
| 56 | CN | CH₃N- | 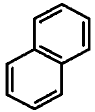 | 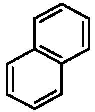 |
| 57 | 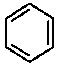 | S | 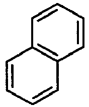 | 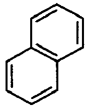 |
| 58 | 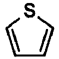 | S | 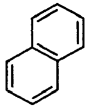 | 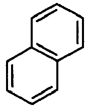 |
| 59 | 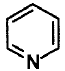 | O | 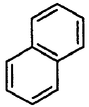 | 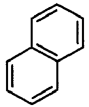 |
| 60 | 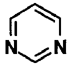 | O | 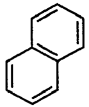 | 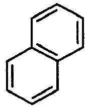 |
| 61 | 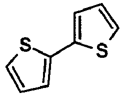 | O | 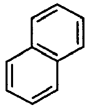 | 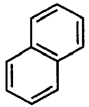 |
| 62 | 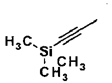 | O | 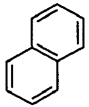 | 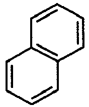 |
| 63 | 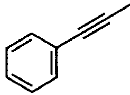 | O | 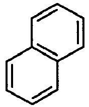 | 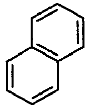 |
| 64 | Cl | O | 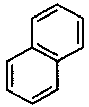 | 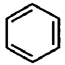 |
| 65 | Cl | O | 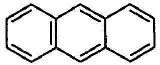 | 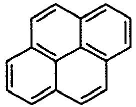 |
| 66 | Cl | S | 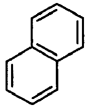 | 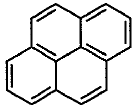 |
| 67 | Cl | O | 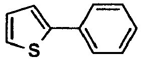 | 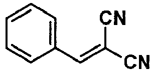 |
| 68 | Cl | O | 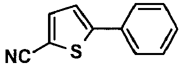 | 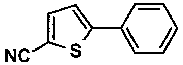 |
| 69 | Cl | O | 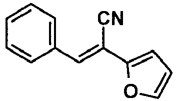 | 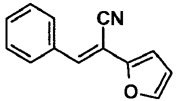 |
| 70 | Cl | O | 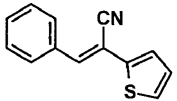 | 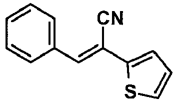 |
| 71 | Cl | O | 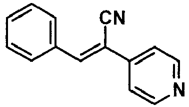 | 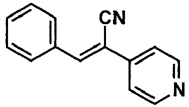 |
| 72 | H | O | 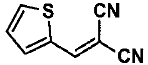 | 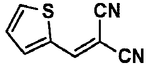 |
| 73 | Cl | O | 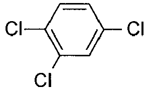 | 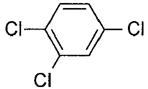 |
| 74 | Cl | O | 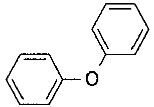 | 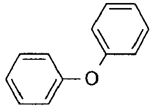 |
| 75 | Cl | O | 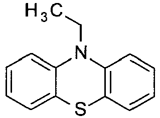 | 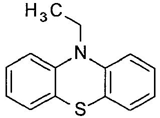 |
| 76 | Cl | O | 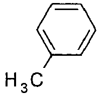 | 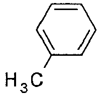 |
| 77 | Cl | O | 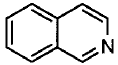 | 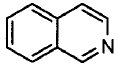 |
| 78 | Cl | O | 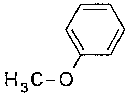 | 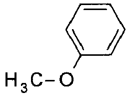 |
| 79 | Cl | O | 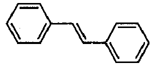 | 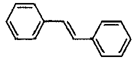 |
| 80 | Cl | O | 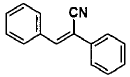 | 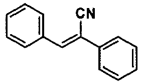 |
| 81 | Cl | 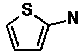 | 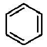 | 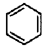 |
| 82 | Cl | 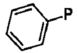 | 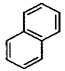 | 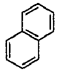 |
| 83 | Cl | Se | 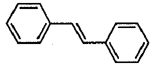 | 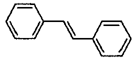 |
| 84 | Cl | Te | 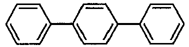 | 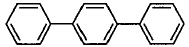 |

### Preparation example of an inverted BHJ-cell:

An inverted BHJ-cell was prepared with compound **1** as an example of a donor substance present in the photoactive material of the present invention which is used for preparing the inverted BHJ cell, which is a solar cell of the present invention, and with C₆₀ as an example of an acceptor substance present in the photoactive material of the present invention which is used for preparing the inverted BHJ cell, which is a solar cell of the present invention.

Glass substrates were used as received from Geomatec. ITO as transparent electrode had a thickness of 90 nm. The specific resistivity was 15 Ohm/cm. The root-mean-square roughness (RMS) was less than 2 nm.

Before deposition of organic layers, the substrate was ozone treated under UV light for 15 minutes (UV-ozone cleaning). Inverted bulk heterojunction cells (inverted BHJ cells) were produced under high vacuum conditions (pressure approx. 2x10⁻⁶ mbar).

### ITO/n-C₆₀/C₆₀/Compound 1:C₆₀//BPAPF/p-BPAPF/NDP-9/Al:

The inverted BHJ cell was produced by covaporization of compound 1 with C₆₀ onto the ITO substrate. The vapor deposition rate for both layers was 0.2 nm/second. The weight ratio of compound 1 to C₆₀ was 1:1. Before and after, the doped ETL and HTL layers were applied by vapor deposition. Finally, an Al layer was applied by vapor deposition as a top electrode. The vaporization temperature of the inventive compound was 260°C. The cell had an area of 0.275 cm².

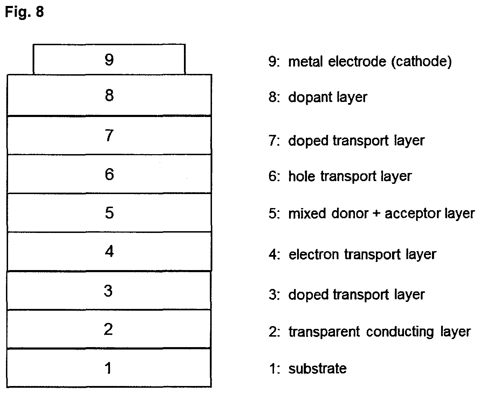

The device setup of the prepared inverted BHJ-cell according to the invention is shown in figure 8. According to the nomenclature of figure 8, the respective layers of the prepared inverted BHJ-cell are denoted as follows:

| | | |
|---|---|---|
| 1: | substrate | glass |
| 2: | transparent conducting layer | ITO |
| 3: | doped transport layer | n-C₆₀ |
| 4: | electron transport layer | C₆₀ |
| 5: | mixed donor + acceptor layer | Compound **1**:C₆₀ |
| 6: | hole transport layer | BPAPF |
| 7: | doped transport layer | p-BPAPF |
| 8: | dopant layer | NDP-9 |
| 9: | metal electrode (cathode) | Al |

| | | |
|---|---|---|
| BPAPF: 9,9-bis[4-(N,N-bis-biphenyl-4-yl-amino)phenyl]-9H-fluorene, CAS No: 174141-92-5 NDP-9: Novaled Dopant P-Side No. 9 | | |

The thickness oft he respective layers is as follows:

| | |
|---|---|
| n-C₆₀ | 10 nm |
| C₆₀ | 5 nm |
| Compound **1**:C₆₀ | 50 nm |
| BPAPF | 5 nm |
| p-BPAPF | 20 nm |
| NDP-9 | 5 nm |
| Al | 100 nm |

### Measurements:

The solar simulator used was an AM 1.5 Simulator from L.O.T. Oriel with a 300 W xenon lamp (modell LSH201). The current-voltage-characteristics were measured under ambient conditions with a Keithley 2400 series. The intensity of the solar simulator was calibrated with a monocrystalline solar cell (Fraunhofer ISE) and the deviation factor was determined to be approximately 1. Results are shown in the following Table 1.

**Table 1:**

| Cell | I_{SC} [mA/cm²] | V_{OC} [mV] | FF [%] | η [%] |
|---|---|---|---|---|
| 50nm compound **1**:C₆₀ (1:1) 20nm BPAPF; RT | 6,7 | -920 | 47 | 2,9 |
| 50nm compound **1**:C₆₀ (1:1) 20nm BPAPF, 60°C | 7,0 | -880 | 52 | 3,2 |

| | | | | |
|---|---|---|---|---|
| η efficiency FF fill factor I_{sc} short-circuit current V_{OC} open-circuit voltage RT room temperature = 20°C | | | | |

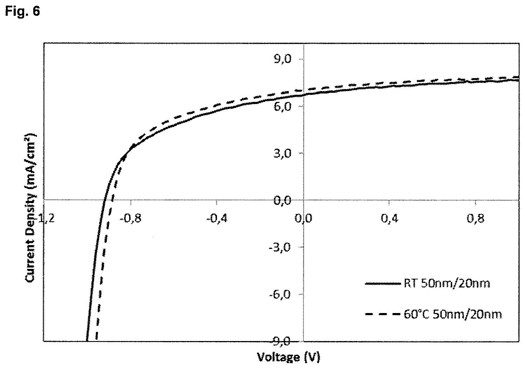

Figure 6 shows the current density-versus-voltage (J-V) curve for the above-decribed inverted BHJ-cell according to the present invention.

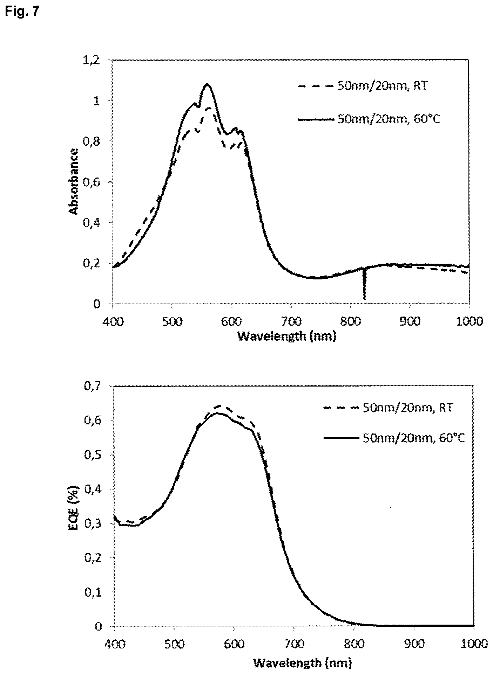

Figure 7 shows the absorption spectrum and the External Quantum Efficiency (EQE) spectrum of compound 1 according to formula (I).

Similar inverted BHJ cell were produced using compounds **26, 27** and **79.** The respective measurement results are presented in Tables 2, 3 and 4:

**Table 2:**

| Cell | I_{SC} [mA/cm²] | V_{OC} [mV] | FF [%] | η [%] |
|---|---|---|---|---|
| 50nm compound **26**:C₆₀ (1:1) 20nm BPAPF; 70°C | 6,1 | -1000 | 46 | 2,8 |

**Table 3:**

| Cell | I_{SC} [mA/cm²] | V_{OC} [mV] | FF [%] | η [%] |
|---|---|---|---|---|
| 30nm compound **27**:C₆₀ (1:1) 20nm BPAPF; 70°C | 6,5 | -680 | 42 | 1,9 |

**Table 4:**

| Cell | I_{SC} [mA/cm²] | V_{OC} [mV] | FF [%] | η [%] |
|---|---|---|---|---|
| 30nm compound **79**:C₆₀ (1:1) 20nm BPAPF; 70°C | 7,1 | -1000 | 47 | 3,3 |

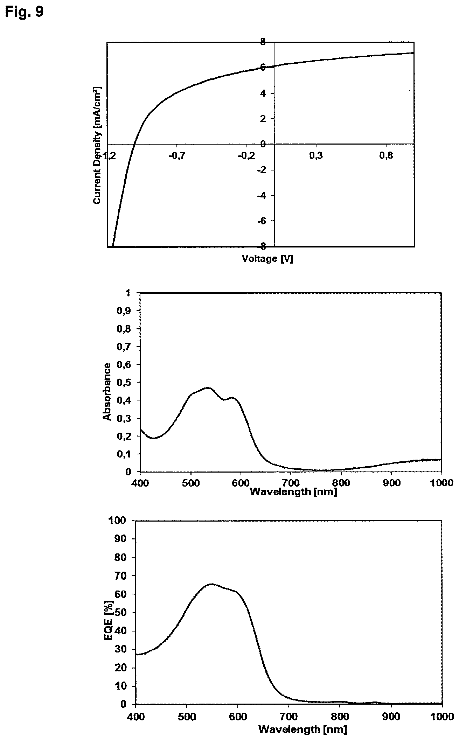

Figure 9 shows the current density-versus-voltage (J-V) curve, the absorption spectrum and the External Quantum Efficiency (EQE) spectrum of compound **26** for an inverted BHJ-cell according to the present invention.

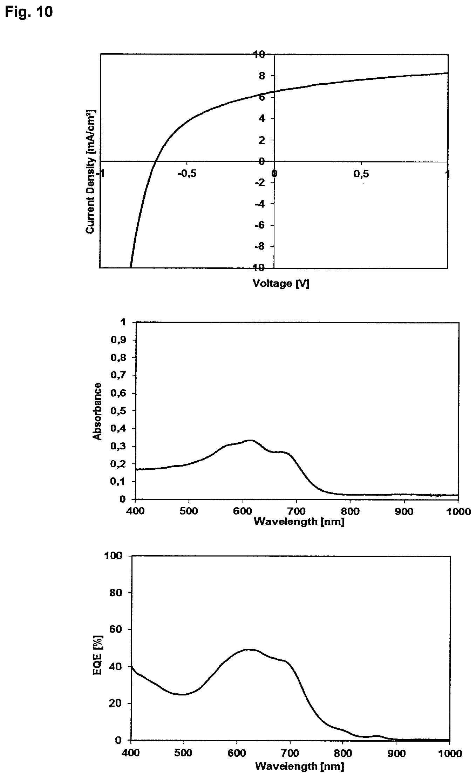

Figure 10 shows the current density-versus-voltage (J-V) curve, the absorption spectrum and the External Quantum Efficiency (EQE) spectrum of compound **27** for an inverted BHJ-cell according to the present invention.

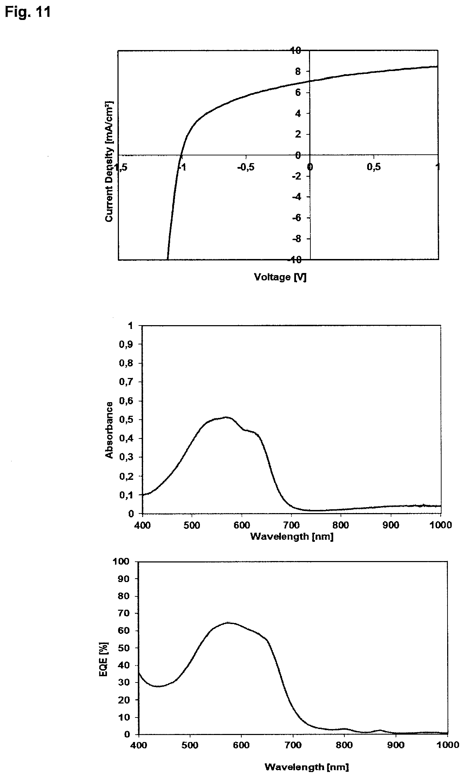

Figure 11 shows the current density-versus-voltage (J-V) curve, the absorption spectrum and the External Quantum Efficiency (EQE) spectrum of compound **79** for an inverted BHJ-cell according to the present invention.

## Claims

1. A photoactive material comprising a donor substance and an acceptor substance, wherein
**a.** the donor substance comprises or consists of one or more compounds of formula (I), or
**b.** the acceptor substance comprises or consists of one or more compounds of formula (I), or
**c.** the donor substance comprises or consists of a first compound of formula (I) and the acceptor substance comprises a second compound of formula (I) with the proviso that the first and second compound are not the same, 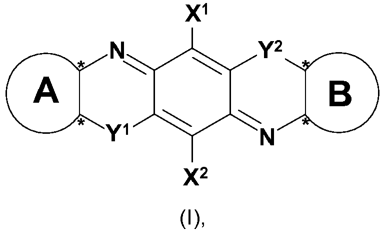 wherein in formula (I)
each of A and B independently of each other indicates a 5- or 6-membered ring which includes the carbon atoms marked with *, respectively,
wherein each of the rings A and B is independently of each other
(i) aromatic or heteroaromatic,
(ii) unsubstituted or substituted,
and is
(iii) not annealed or annealed with one or more further rings not including carbon atoms marked with *, respectively, or is connected with one or more further rings by a single common atom to give a spiro compound,
X¹, X² are independently of each other selected from the group consisting of hydrogen and substituents,
Y¹, Y² are independently of each other selected from the group consisting of O, S, NR¹, PR¹, Se and Te,
wherein
each R¹ is independently selected from the group consisting of H, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl, **characterised in that** in formula (I) A or B or each of A and B independently of each other indicates a 5 or 6-membered ring present in a moiety selected from the group consisting of 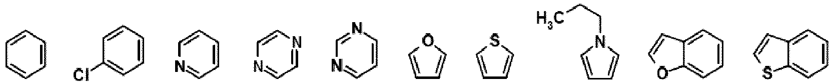 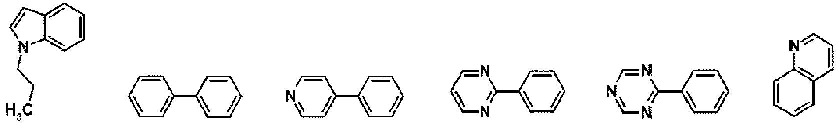 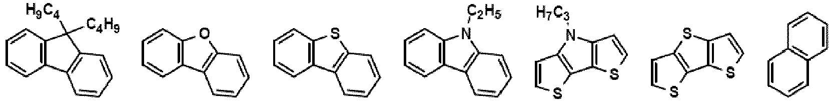 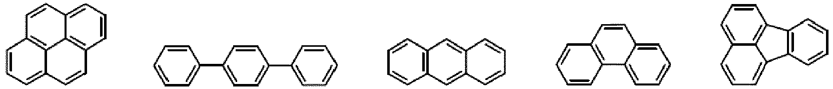 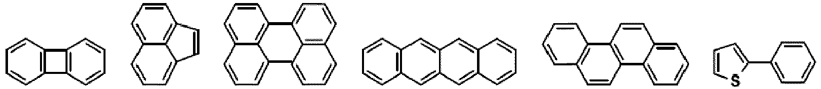 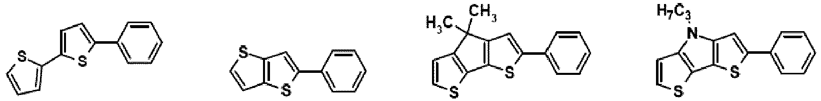 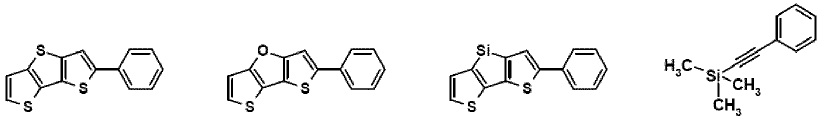 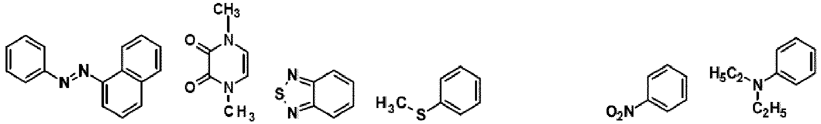 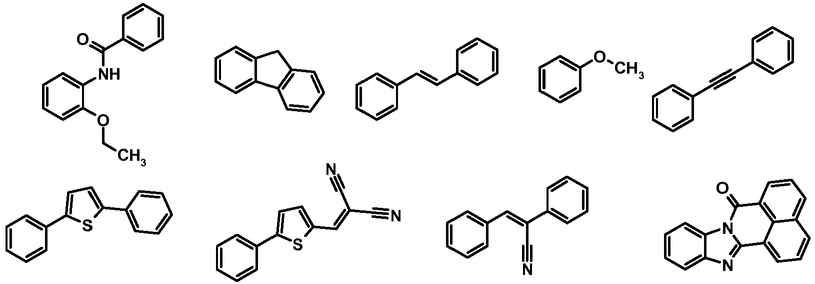 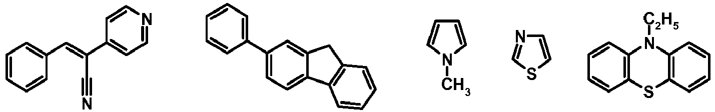
wherein in each 5 or 6-membered ring present in said moieties and independently of each other indicating A or B, respectively, two adjacent carbon atoms are identical with carbon atoms marked with * in formula (I),
wherein the donor substance and the acceptor substance are part of respective layers of a donor-acceptor bilayer or part of a donor-acceptor mixed layer (23 - Figure 3, 24 - Figure 4).

2. The photoactive material according to claim 1, wherein each of the rings A and B is independently of each other
(iii) annealed with one or more further unsubstituted or substituted rings not including carbon atoms marked with *, respectively, wherein each of said one or more further rings is independently of each other non-aromatic, aromatic or heteroaromatic.

3. The photoactive material according to any of claims 1 to 2, wherein X¹, X² are independently of each other selected from the group consisting of H, D, F, Cl, Br, I, NO₂, CN, OH, C(O)OR¹, OC(O)OR¹, NR¹C(O)NR²R³, C(O)NR²R³, S(O)R¹, SO₂R¹, SO₃R¹, OSO₃R¹, COR¹, SiR¹R²R³, P(O)R¹R², P(O)OR¹OR², OR¹, SR¹, NR²R³, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl,
wherein
(i) R¹, R², R³ are independently of each other selected from the group consisting of H, unsubstituted alkyl having 1 to 24 carbon atoms, substituted alkyl having 1 to 24 carbon atoms including substituents, unsubstituted alkenyl having 2 to 18 carbon atoms, substituted alkenyl having 2 to 18 carbon atoms including substituents, unsubstituted alkynyl having 2 to 18 carbon atoms, substituted alkynyl having 2 to 18 carbon atoms including substituents, unsubstituted cycloalkyl having 3 to 8 carbon atoms, substituted cycloalkyl having 3 to 8 carbon atoms including substituents, unsubstituted aryl, substituted aryl, unsubstituted heteroaryl, and substituted heteroaryl;
or
(ii) R¹ has the meaning as defined under (i), and
R² and R³ together and in combination with the atom connecting R² and R³ constitute an aromatic or heteroaromatic or aliphatic 5 to 8-membered ring.

4. The photoactive material according to claim 3, wherein in formula (I) X¹, X² are independently of each other selected from the group consisting of H, F, Cl, Br, I, NO₂, CN, C(O)OR¹, C(O)NR²R³, SiR¹R²R³, unsubstituted alkyl having 1 to 12 carbon atoms, substituted alkyl having 1 to 12 carbon atoms including substituents, unsubstituted aryl having 6 to 18 carbon atoms, substituted aryl having 6 to 18 carbon atoms including substituents, unsubstituted heteroaryl having 4 to 12 carbon atoms, and substituted heteroaryl having 4 to 12 carbon atoms including substituents.

5. The photoactive material according to any of claims 1 to 4, wherein in formula (I) A and B are independently selected from the group consisting of unsubstituted aryl having 5 to 30 carbon atoms, substituted aryl having 5 to 30 carbon atoms including substituents, unsubstituted heteroaryl having 5 to 28 carbon atoms, substituted heteroaryl having 5 to 28 carbon atoms including substituents; wherein any substituent, if present, is a substituent Z¹, where Z¹ is independently of each other selected from the group consisting of unsubstituted alkyl having 1 to 24 carbon atoms, substituted alkyl having 1 to 24 carbon atoms including substituents, unsubstituted cycloalkyl having 3 to 8 carbon atoms, substituted cycloalkyl having 3 to 8 carbon atoms including substituents, F, Cl, Br, I, OR⁴, SR⁴, NR⁵R⁶, CN, NO₂, C(O)R⁷, N=N-R⁵, SiR⁸R⁹R¹⁰, C=C-R¹¹, unsubstituted aryl having 6 to 20 carbon atoms, substituted aryl having 6 to 20 carbon atoms including substituents, unsubstituted heteroaryl having 4 to 12 carbon atoms, and W;
wherein R⁴, R⁵, R⁶ are independently of each other selected from the group consisting of H, alkyl having 1 to 8 carbon atoms, and aryl having 6 to 10 carbon atoms;
R⁷, R⁸, R⁹, R¹⁰ are independently of each other selected from the group consisting of H, OH, alkyl having 1 to 6 carbon atoms, aryl having 6 to 10 carbon atoms, O-alkyl having 1 to 8 carbon atoms, and O-aryl having 6 to 10 carbon atoms;
R¹¹ is independently of each other selected from the group consisting of aryl having 6 to 10 carbon atoms, heteroaryl having 4 to 10 carbon atoms, Si(alkyl having 1 to 6 carbon atoms)₃, and SiPh₃;
W is 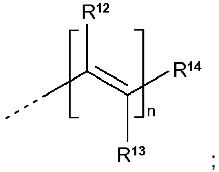
wherein n is selected from the group consisting of 1, 2, 3 and 4;
R¹², R¹³, R¹⁴ are independently of each other selected from the group consisting of H, CN, alkyl having from 1 to 8 carbon atoms, aryl having from 6 to 10 carbon atoms, and heteroaryl having from 4 to 10 carbon atoms.

6. The photoactive material according to any of claims 1 to 5, wherein in formula (I) X¹, X² are independently of each other selected from the group consisting of H, F, and Cl; and/or wherein Y¹, Y² are independently of each other selected from the group consisting of O and S.

7. The photoactive material according to any of claims 1 to 6, wherein in formula (I) A and B are independently of each other selected from the group consisting of phenyl, substituted phenyl, naphthyl, substituted naphthyl, biphenyl, substituted biphenyl, terphenyl, substituted terphenyl, anthranyl, substituted anthranyl, pyrenyl, substituted pyrenyl, perylenyl, substituted perylenyl, thienyl, substituted thienyl, pyridyl, substituted pyridyl, quinolinyl, substituted quinolinyl, bithienyl, substituted bithienyl, terthienyl, substituted terthienly, wherein any substituent, if present, is a substituent Z², where each Z² is independently of each other selected from the group consisting of alkyl having 1 to 12 carbon atoms, O(alkyl) having 1 to 6 carbon atoms, F, Cl, CN, dicyanovinyl, cyanopyridylvinyl, cyanophenylvinyl, phenyl, naphthyl, thienyl, bithienyl, and pyridyl.

8. The photoactive material according to any of claims 1 to 7, wherein the photoactive material further comprises one or more semiconductor material(s).

9. The photoactive material according to any of claims 1 to 8, wherein the donor substance comprises or consists of one or more compounds of formula (I) as defined in any of claims 1 to 8, and wherein the acceptor substance comprises or consists of one or more compounds selected from the group consisting of
(i) fullerenes and fullerene derivatives, preferably selected from the group consisting of C₆₀, C₇₀ and [6,6]-phenyl-C₆₁-butyric acid methyl ester, and
(ii) 3,4,9,10-perylenetetracarboxyl-bisbenzimidazole (PTCBI).

10. An organic solar cell or photodetector **characterised in that** it comprises a photoactive material according to any of claims 1 to 9.

11. A photoelectric conversion device comprising or consisting of two or more organic solar cells according to claim 10.

12. The photoelectric conversion device according to claim 11, wherein the organic solar cells are arranged as tandem cells or as inverted tandem cells.

13. A compound of formula (I) as defined in any of claims 1 to 7 for use as donor substance or as acceptor substance in a photoactive material, wherein the photoactive material is part of a layer of a donor-acceptor bilayer or part of a donor-acceptor mixed layer (23 - Figure 3, 24 - Figure 4).

14. Use of a compound of formula (I) as defined in any of claims 1 to 7 as donor substance or as acceptor substance in a photoactive material, wherein the photoactive material is part of a layer of a donor-acceptor bilayer or part of a donor-acceptor mixed layer (23 - Figure 3, 24 - Figure 4).

## Patentansprüche

1. Photoaktives Material, umfassend eine Donor-Substanz und eine Akzeptor-Substanz, wobei:
a. die Donor-Substanz eine oder mehrere Verbindungen mit der Formel (I) umfasst oder daraus besteht, oder
b. die Akzeptor-Substanz eine oder mehrere Verbindungen mit der Formel (I) umfasst oder daraus besteht, oder
c. die Donor-Substanz eine erste Verbindung mit der Formel (I) umfasst oder daraus besteht, und die Akzeptor-Substanz eine zweite Verbindung mit der Formel (I) umfasst, mit der Maßgabe, dass die erste und zweite Verbindung nicht dieselbe sind, 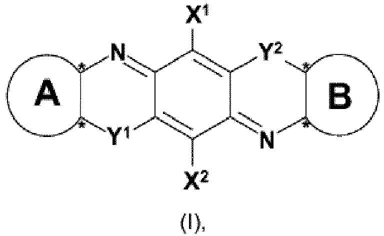 wobei in der Formel (I):
jedes von A und B unabhängig voneinander einen 5- oder 6-gliedrigen Ring anzeigt, der die jeweils mit * markierten Kohlenstoffatome aufweist,
wobei jeder der Ringe A und B unabhängig voneinander ist:
(i) aromatisch oder heteroaromatisch,
(ii) unsubstituiert oder substituiert,
und ist:
(iii) nicht anelliert oder anelliert mit einem oder mehreren weiteren Ringen, die jeweils keine mit * markierten Kohlenstoffatome aufweisen, oder verbunden ist mit einem oder mehreren weiteren Ringen durch ein einzelnes gemeinsames Atom, um eine Spiro-Verbindung zu ergeben,
X¹, X² unabhängig voneinander ausgewählt sind aus der Gruppe bestehend aus Wasserstoff und Substituenten,
Y¹, Y² unabhängig voneinander ausgewählt sind aus der Gruppe bestehend aus O, S, NR¹, PR¹, Se und Te,
wobei:
jedes R¹ unabhängig ausgewählt ist aus der Gruppe bestehend aus H, unsubstituiertem Alkyl mit 1 bis 24 Kohlenstoffatomen, substituiertem Alkyl mit 1 bis 24 Kohlenstoffatomen, umfassend Substituenten, unsubstituiertem Alkenyl mit 2 bis 18 Kohlenstoffatomen, substituiertem Alkenyl mit 2 bis 18 Kohlenstoffatomen, umfassend Substituenten, unsubstituiertem Alkylnyl mit 2 bis 18 Kohlenstoffatom, substituiertem Alkynyl mit 2 bis 18 Kohlenstoffatomen, umfassend Substituenten, unsubstituiertem Cycloalkyl mit 3 bis 8 Kohlenstoffatomen, substituiertem Cycloalkyl mit 3 bis 8 Kohlenstoffatomen, umfassend Substituenten, unsubstituiertem Aryl, substituiertem Aryl, unsubstituiertem Heteroaryl und substituiertem Heteroaryl,
**dadurch gekennzeichnet, dass** in der Formel (I) A oder B oder jedes von A und B unabhängig voneinander einen 5- oder 6-gliedrigen Ring anzeigt, der in einer Einheit vorhanden ist, die ausgewählt ist aus der Gruppe bestehend aus: 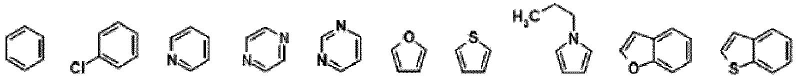 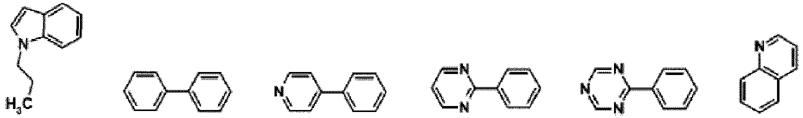 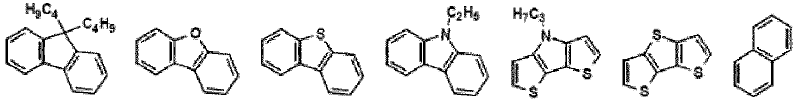 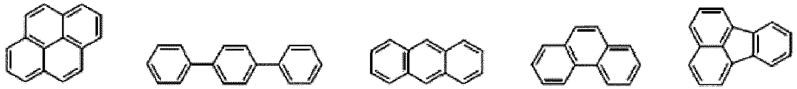 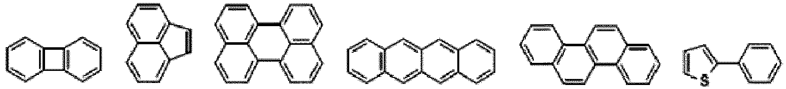 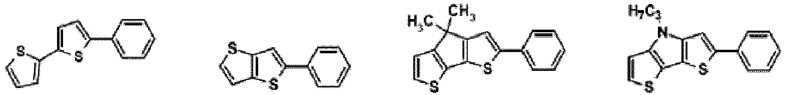 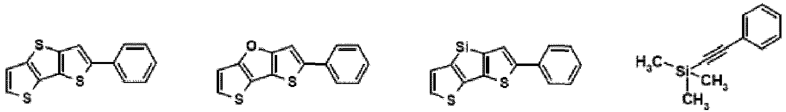 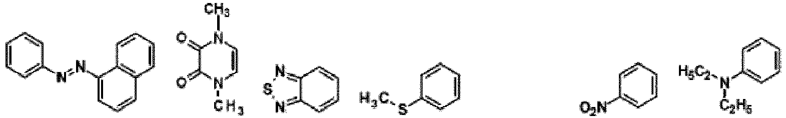 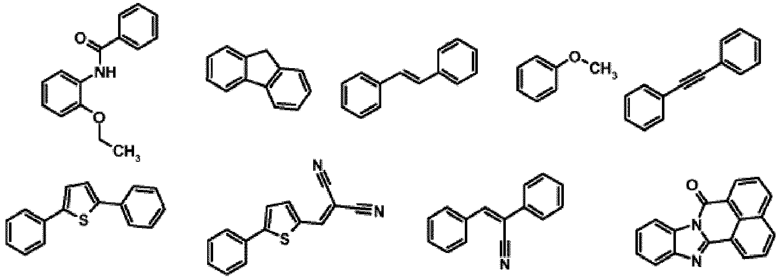 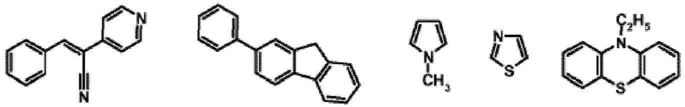
wobei in jedem 5- oder 6-gliedrigen Ring in den Einheiten und unabhängig voneinander, die jeweils A oder B anzeigen, zwei benachbarte Kohlenstoffatome mit Kohlenstoffatomen identisch sind, die mit * in der Formel (I) markiert sind,
wobei die Donor-Substanz und die Akzeptor-Substanz Teil jeweiliger Schichten einer Donor-Akzeptor-Doppelschicht oder Teil einer Donor-Akzeptor-Mischschicht sind (23 - Figur 3, 24 - Figur 4).

2. Photoaktives Material nach Anspruch 1, wobei jeder der Ringe A und B unabhängig voneinander
(iii) mit einem oder mehreren weiteren unsubstituierten oder substituierten Ringen anelliert ist, nicht umfassend Kohlenstoffatome, die jeweils mit * markiert sind, wobei jeder von dem einen oder den mehreren weiteren Ringen unabhängig voneinander nicht-aromatisch, aromatisch oder heteroaromatisch ist.

3. Photoaktives Material nach einem der Ansprüche 1 bis 2, wobei X¹, X² unabhängig voneinander ausgewählt sind aus der Gruppe bestehend aus H, D, F, Cl, Br, I, NO₂, CN, OH, C(O)OR¹, OC(O)OR¹, NR¹C(O)NR²R³, C(O)NR²R³, S(O)R¹, SO₂R¹, SO₃R¹, OSO₃R¹, COR¹, SiR¹R²R³, P(O)R¹R², P(O)OR¹OR², OR¹, SR¹, NR²R³, unsubstituiertem Alkyl mit 1 bis 24 Kohlenstoffatomen, substituiertem Alkyl mit 1 bis 24 Kohlenstoffatomen, umfassend Substituenten, unsubstituiertem Alkenyl mit 2 bis 18 Kohlenstoffatomen, substituiertem Alkenyl mit 2 bis 18 Kohlenstoffatomen, umfassend Substituenten, unsubstituiertem Alkynyl mit 2 bis 18 Kohlenstoffatomen, substituiertem Alkynyl mit 2 bis 18 Kohlenstoffatomen, umfassend Substituenten, unsubstituiertem Cycloalkyl mit 3 bis 8 Kohlenstoffatomen, substituiertem Cycloalkyl mit 3 bis 8 Kohlenstoffatomen, umfassend Substituenten, unsubstituiertem Aryl, substituiertem Aryl, unsubstituiertem Heteroaryl und substituiertem Heteroaryl,
wobei:
(i) R¹, R², R³ unabhängig voneinander ausgewählt sind aus der Gruppe bestehend aus H, unsubstituiertem Alkyl mit 1 bis 24 Kohlenstoffatomen, substituiertem Alkyl mit 1 bis 24 Kohlenstoffatomen, umfassend Substituenten, unsubstituiertem Alkenyl mit 2 bis 18 Kohlenstoffatomen, substituiertem Alkenyl mit 2 bis 18 Kohlenstoffatomen, umfassend Substituenten, unsubstituiertem Alkynyl mit 2 bis 18 Kohlenstoffatomen, substituiertem Alkynyl mit 2 bis 18 Kohlenstoffatomen, umfassend Substituenten, unsubstituiertem Cycloalkyl mit 3 bis 8 Kohlenstoffatomen, substituiertem Cycloalkyl mit 3 bis 8 Kohlenstoffatomen, umfassend Substituenten, unsubstituiertem Aryl, substituiertem Aryl, unsubstituiertem Heteroaryl und substituiertem Heteroaryl;
oder
(ii) R¹ die Bedeutung wie unter (i) definiert hat, und
R² und R³ gemeinsam und in Kombination mit dem Atom, das R² und R³ verbindet, einen aromatischen oder heteroaromatischen oder aliphatischen 5- bis 8-gliedrigen Ring darstellen.

4. Photoaktives Material nach Anspruch 3, wobei in der Formel (I) X¹, X² unabhängig voneinander ausgewählt sind aus der Gruppe bestehend aus H, F, Cl, Br, I, NO₂, CN, C(O)OR¹, C(O)NR²R³, SiR¹R²R³, unsubstituiertem Alkyl mit 1 bis 12 Kohlenstoffatomen, substituiertem Alkyl mit 1 bis 12 Kohlenstoffatomen, umfassend Substituenten, unsubstituiertem Aryl mit 6 bis 18 Kohlenstoffatomen, substituiertem Aryl mit 6 bis 18 Kohlenstoffatomen, umfassend Substituenten, unsubstituiertem Heteroaryl mit 4 bis 12 Kohlenstoffatomen und substituiertem Heteroaryl mit 4 bis 12 Kohlenstoffatomen, umfassend Substituenten.

5. Photoaktives Material nach einem der Ansprüche 1 bis 4, wobei in der Formel (I) A und B unabhängig ausgewählt sind aus der Gruppe bestehend aus unsubstituiertem Aryl mit 5 bis 30 Kohlenstoffatomen, substituiertem Aryl mit 5 bis 30 Kohlenstoffatomen, umfassend Substituenten, unsubstituiertem Heteroaryl mit 5 bis 28 Kohlenstoffatomen, substituiertem Heteroaryl mit 5 bis 28 Kohlenstoffatomen, umfassend Substituenten; wobei irgendein Substituent, wenn vorhanden, ein Substituent Z¹ ist, wobei Z¹ unabhängig voneinander ausgewählt ist aus der Gruppe bestehend aus unsubstituiertem Alkyl mit 1 bis 24 Kohlenstoffatomen, substituiertem Alkyl mit 1 bis 24 Kohlenstoffatomen, umfassend Substituenten, unsubstituiertem Cycloalkyl mit 3 bis 8 Kohlenstoffatomen, substituiertem Cycloalkyl mit 3 bis 8 Kohlenstoffatomen, umfassend Substituenten, F, Cl, Br, I, OR⁴, SR⁴, NR⁵R⁶, CN, NO₂, C(O)R⁷, N=N-R⁵, SiR⁸R⁹R¹⁰, C≡C-R¹¹, unsubstituiertem Aryl mit 6 bis 20 Kohlenstoffatomen, substituiertem Aryl mit 6 bis 20 Kohlenstoffatomen, umfassend Substituenten, unsubstituiertem Heteroaryl mit 4 bis 12 Kohlenstoffatomen, und W;
wobei R⁴, R⁵, R⁶ unabhängig voneinander ausgewählt sind aus der Gruppe bestehend aus H, Alkyl mit 1 bis 8 Kohlenstoffatomen und Aryl mit 6 bis 10 Kohlenstoffatomen;
R⁷, R⁸, R⁹, R¹⁰ unabhängig voneinander ausgewählt sind aus der Gruppe bestehend aus H, OH, Alkyl mit 1 bis 6 Kohlenstoffatomen, Aryl mit 6 bis 10 Kohlenstoffatomen, O-Alkyl mit 1 bis 8 Kohlenstoffatomen und O-Aryl mit 6 bis 10 Kohlenstoffatomen;
R¹¹ unabhängig voneinander ausgewählt ist aus der Gruppe bestehend aus Aryl mit 6 bis 10 Kohlenstoffatomen, Heteroaryl mit 4 bis 10 Kohlenstoffatomen, Si (alkyl mit 1 bis 6 Kohlenstoffatomen)₃ und SiPh₃;
W ist 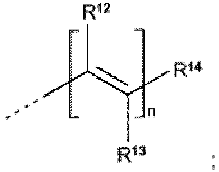
wobei n ausgewählt ist aus der Gruppe bestehend aus 1, 2, 3 und 4;
R¹², R¹³, R¹⁴ unabhängig voneinander ausgewählt sind aus der Gruppe bestehend aus H, CN, Alkyl mit 1 bis 8 Kohlenstoffatomen, Aryl mit 6 bis 10 Kohlenstoffatomen und Heteroaryl mit 4 bis 10 Kohlenstoffatomen.

6. Photoaktives Material nach einem der Ansprüche 1 bis 5, wobei in der Formel (I) X¹, X² unabhängig voneinander ausgewählt sind aus der Gruppe bestehend aus H, F, Cl; und/oder wobei Y¹, Y² unabhängig voneinander ausgewählt sind aus der Gruppe bestehend aus O und S.

7. Photoaktives Material nach einem der Ansprüche 1 bis 6, wobei in der Formel (I) A und B unabhängig voneinander ausgewählt sind aus der Gruppe bestehend aus Phenyl, substituiertem Phenyl, Naphthyl, substituiertem Naphthyl, Biphenyl, substituiertem Biphenyl, Terphenyl, substituiertem Terphenyl, Anthranyl, substituiertem Anthranyl, Pyrenyl, substituiertem Pyrenyl, Perylenyl, substituiertem Perylenyl, Thienyl, substituiertem Thienyl, Pyridyl, substituiertem Pyridyl, Chinolinyl, substituiertem Chinolinyl, Bithienyl, substituiertem Bithienyl, Terthienyl, substituiertem Terthienyl, wobei irgendein Substituent, wenn vorhanden, ein Substituent Z² ist, wobei Z² unabhängig voneinander ausgewählt ist aus der Gruppe bestehend aus Alkyl mit 1 bis 12 Kohlenstoffatomen, O(alkyl) mit 1 bis 6 Kohlenstoffatomen, F, Cl, CN, Dicyanovinyl, Cyanopyridylvinyl, Cyanophenylvinyl, Phenyl, Naphthyl, Thienyl, Bithienyl und Pyridyl.

8. Photoaktives Material nach einem der Ansprüche 1 bis 7, wobei das photoaktive Material ferner ein oder mehrere Halbleitermaterialien umfasst.

9. Photoaktives Material nach einem der Ansprüche 1 bis 8, wobei die Donor-Substanz eine oder mehrere Verbindungen mit der Formel (I) umfasst oder daraus besteht, wie in einem der Ansprüche 1 bis 8 definiert, und wobei die Akzeptor-Substanz eine oder mehrere Verbindungen umfasst oder daraus besteht, die ausgewählt sind aus der Gruppe bestehend aus:
(i) Fullerenen und Fulleren-Derivaten, die vorzugsweise ausgewählt sind aus der Gruppe bestehend aus C₆₀, C₇₀ und [6,6]-Phenyl-C₆₁-buttersäuremethylester, und
(ii) 3,4,9,10-Perylentetracarboxyl-bisbenzimidazol (PTCBI).

10. Organische Solarzelle oder Photodetektor, **dadurch gekennzeichnet, dass** diese oder dieser ein photoaktives Material nach einem der Ansprüche 1 bis 9 umfasst.

11. Photoelektrische Wandlervorrichtung, umfassend zwei oder mehr organische Solarzellen nach Anspruch 10 oder bestehend daraus.

12. Photoelektrische Wandlervorrichtung nach Anspruch 11, wobei die organischen Solarzellen als Tandemzellen oder als invertierte Tandemzellen angeordnet sind.

13. Verbindung mit der Formel (I), wie in einem der Ansprüche 1 bis 7 definiert, zur Verwendung als Donor-Substanz oder als Akzeptor-Substanz in einem photoaktiven Material, wobei das photoaktive Material Teil einer Schicht einer Donor-Akzeptor-Doppelschicht oder Teil einer Donor-Akzeptor-Mischschicht ist (23 - Figur 3, 24 - Figur 4).

14. Verwendung einer mit der Formel (I), wie in einem der Ansprüche 1 bis 7 definiert, als Donor-Substanz oder als Akzeptor-Substanz in einem photoaktiven Material, wobei das photoaktive Material Teil einer Schicht einer Donor-Akzeptor-Doppelschicht oder Teil einer Donor-Akzeptor-Mischschicht ist (23 - Figur 3, 24 - Figur 4).

## Revendications

1. Matériau photoactif comprenant une substance donneuse et une substance accepteuse, dans lequel
a. la substance donneuse comprend ou est constituée d'un ou plusieurs composés de formule (I), ou
b. la substance accepteuse comprend ou est constituée d'un ou plusieurs composés de formule (I), ou
c. la substance donneuse comprend ou est constituée d'un premier composé de formule (I) et la substance accepteuse comprend un deuxième composé de formule (I) à condition que les premier et deuxième composés ne soient pas identiques, 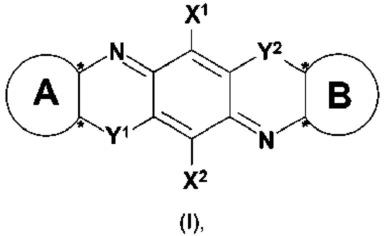 dans lequel, dans la formule (I)
chacun de A et B, indépendamment l'un de l'autre, désigne un cycle de 5 ou 6 chaînons qui comprend les atomes de carbone marqués avec *, respectivement,
dans lequel chacun des cycles A et B est, indépendamment l'un de l'autre,
(i) aromatique ou hétéroaromatique,
(ii) non substitué ou substitué,
et est
(iii) non annelé ou annelé avec un ou plusieurs cycles supplémentaires ne comprenant pas les atomes de carbone marqués avec *, respectivement, ou est relié à un ou plusieurs cycles supplémentaires par un atome commun unique pour obtenir un composé spiro,
X¹, X² sont, indépendamment l'un de l'autre, choisis dans le groupe constitué d'hydrogène et de substituants, Y¹, Y² sont, indépendamment l'un de l'autre, choisis dans le groupe constitué de O, S, NR¹, PR¹, Se et Te,
dans lequel
chaque R¹ est indépendamment choisi dans le groupe constitué de H, alkyle non substitué ayant 1 à 24 atomes de carbone, alkyle substitué ayant 1 à 24 atomes de carbone comprenant des substituants, alcényle non substitué ayant 2 à 18 atomes de carbone, alcényle substitué ayant 2 à 18 atomes de carbone comprenant des substituants, alcynyle non substitué ayant 2 à 18 atomes de carbone, alcynyle substitué ayant 2 à 18 atomes de carbone comprenant des substituants, cycloalkyle non substitué ayant 3 à 8 atomes de carbone, cycloalkyle substitué ayant 3 à 8 atomes de carbone comprenant des substituants, aryle non substitué, aryle substitué, hétéroaryle non substitué, et hétéroaryle substitué, **caractérisé en ce que**, dans la formule (I), A ou B ou chacun de A et B, indépendamment l'un de l'autre, désigne un cycle de 5 ou 6 chaînons présent dans un fragment choisi dans le groupe constitué de 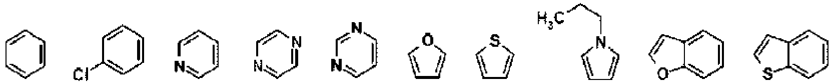 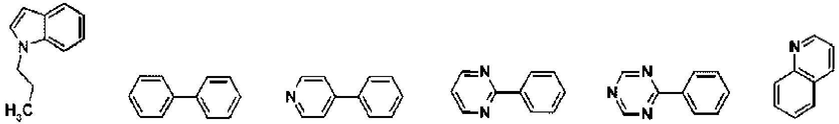 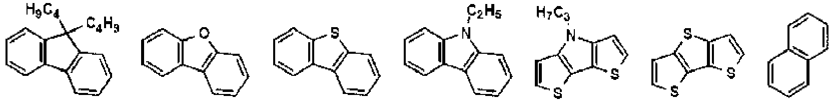 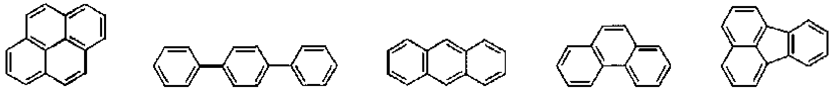 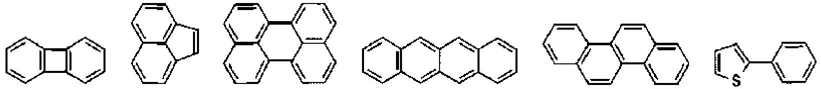 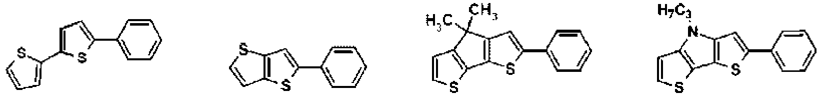 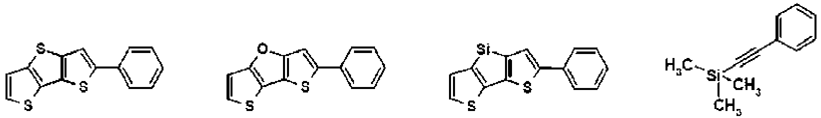 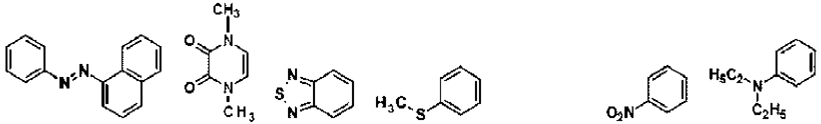 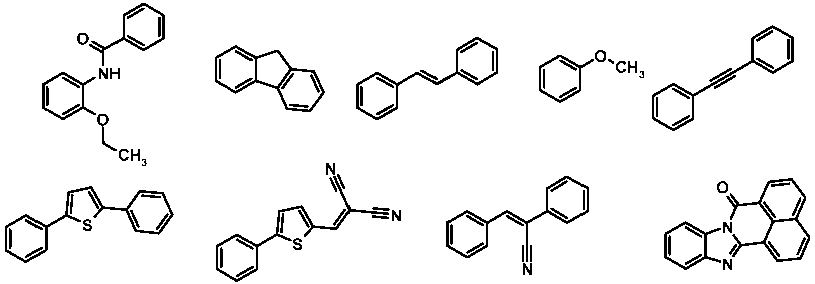 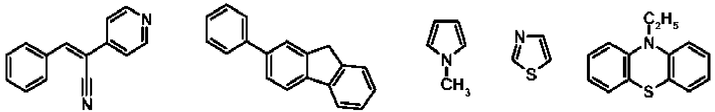
dans lequel, dans chaque cycle de 5 ou 6 chaînons présent dans lesdits fragments et indépendamment des autres désignant A ou B, respectivement, deux atomes de carbone adjacents sont identiques aux atomes de carbone marqués avec * dans la formule (I),
dans lequel la substance donneuse et la substance accepteuse font partie de couches respectives d'une bicouche donneur-accepteur ou partie d'une couche mixte donneur-accepteur (23 - Figure 3, 24 - Figure 4).

2. Matériau photoactif selon la revendication 1, dans lequel chacun des cycles A et B est, indépendamment l'un de l'autre
(iii) annelé avec un ou plusieurs cycles non substitués ou substitués supplémentaires ne comprenant pas des atomes de carbone marqués avec *, respectivement, dans lequel chacun desdits un ou plusieurs cycles supplémentaires est, indépendamment des autres, non aromatique, aromatique ou hétéroaromatique.

3. Matériau photoactif selon l'une quelconque des revendications 1 à 2, dans lequel X¹, X² sont, indépendamment l'un de l'autre, choisis dans le groupe constitué de H, D, F, Cl, Br, I, NO₂, CN, OH, C(O)OR¹, OC(O)OR¹, NR¹C(O)NR²R³, C(O)NR²R³, S(O)R¹, SO₂R¹, SO₃R¹, OSO₃R¹, COR¹, SiR¹R²R³, P(O)R¹R², P(O)OR¹OR², OR¹, SR¹, NR²R³, alkyle non substitué ayant 1 à 24 atomes de carbone, alkyle substitué ayant 1 à 24 atomes de carbone comprenant des substituants, alcényle non substitué ayant 2 à 18 atomes de carbone, alcényle substitué ayant 2 à 18 atomes de carbone comprenant des substituants, alcynyle non substitué ayant 2 à 18 atomes de carbone, alcynyle substitué ayant 2 à 18 atomes de carbone comprenant des substituants, cycloalkyle non substitué ayant 3 à 8 atomes de carbone, cycloalkyle substitué ayant 3 à 8 atomes de carbone comprenant des substituants, aryle non substitué, aryle substitué, hétéroaryle non substitué, et hétéroaryle substitué, dans lequel
(i) R¹, R², R³ sont, indépendamment les uns des autres, choisis dans le groupe constitué de H, alkyle non substitué ayant 1 à 24 atomes de carbone, alkyle substitué ayant 1 à 24 atomes de carbone comprenant des substituants, alcényle non substitué ayant 2 à 18 atomes de carbone, alcényle substitué ayant 2 à 18 atomes de carbone comprenant des substituants, alcynyle non substitué ayant 2 à 18 atomes de carbone, alcynyle substitué ayant 2 à 18 atomes de carbone comprenant des substituants, cycloalkyle non substitué ayant 3 à 8 atomes de carbone, cycloalkyle substitué ayant 3 à 8 atomes de carbone comprenant des substituants, aryle non substitué, aryle substitué, hétéroaryle non substitué, et hétéroaryle substitué ;
ou
(ii) R¹ a la signification telle que définie dans (i), et R² et R³, conjointement et en combinaison avec l'atome reliant R² et R³ constituent un cycle de 5 à 8 chaînons aromatique ou hétéroaromatique ou aliphatique.

4. Matériau photoactif selon la revendication 3, dans lequel, dans la formule (I), X¹, X² sont, indépendamment l'un de l'autre, choisis dans le groupe constitué de H, F, Cl, Br, I, NO₂, CN, C(O)OR¹, C(O)NR²R³, SiR¹R²R³, alkyle non substitué ayant 1 à 12 atomes de carbone, alkyle substitué ayant 1 à 12 atomes de carbone comprenant des substituants, aryle non substitué ayant 6 à 18 atomes de carbone, aryle substitué ayant 6 à 18 atomes de carbone comprenant des substituants, hétéroaryle non substitué ayant 4 à 12 atomes de carbone, et hétéroaryle substitué ayant 4 à 12 atomes de carbone comprenant des substituants.

5. Matériau photoactif selon l'une quelconque des revendications 1 à 4, dans lequel, dans la formule (I), A et B sont indépendamment choisis dans le groupe constitué d'aryle non substitué ayant 5 à 30 atomes de carbone, aryle substitué ayant 5 à 30 atomes de carbone comprenant des substituants, hétéroaryle non substitué ayant 5 à 28 atomes de carbone, hétéroaryle substitué ayant 5 à 28 atomes de carbone comprenant des substituants ; dans lequel un substituant quelconque, le cas échéant, est un substituant Z¹, où Z¹ est, indépendamment des autres, choisi dans le groupe constitué d'alkyle non substitué ayant 1 à 24 atomes de carbone, alkyle substitué ayant 1 à 24 atomes de carbone comprenant des substituants, cycloalkyle non substitué ayant 3 à 8 atomes de carbone, cycloalkyle substitué ayant 3 à 8 atomes de carbone comprenant des substituants, F, Cl, Br, I, OR⁴, SR⁴, NR⁵R⁶, CN, NO₂, C(O)R⁷, N=N-R⁵, SiR⁸R⁹R¹⁰, C≡C-R¹¹, aryle non substitué ayant 6 à 20 atomes de carbone, aryle substitué ayant 6 à 20 atomes de carbone comprenant des substituants, hétéroaryle non substitué ayant 4 à 12 atomes de carbone, et W ;
dans lequel R⁴, R⁵, R⁶ sont, indépendamment les uns des autres, choisis dans le groupe constitué de H, alkyle ayant 1 à 8 atomes de carbone, et aryle ayant 6 à 10 atomes de carbone ;
R⁷, R⁸, R⁹, R¹⁰ sont, indépendamment les uns des autres, choisis dans le groupe constitué de H, OH, alkyle ayant 1 à 6 atomes de carbone, aryle ayant 6 à 10 atomes de carbone, O-alkyle ayant 1 à 8 atomes de carbone, et O-aryle ayant 6 à 10 atomes de carbone ;
R¹¹ est, indépendamment des autres, choisi dans le groupe constitué d'aryle ayant 6 à 10 atomes de carbone, hétéroaryle ayant 4 à 10 atomes de carbone, Si(alkyle ayant 1 à 6 atomes de carbone)₃, et SiPh₃ ;
W est 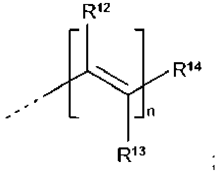
dans lequel n est choisi dans le groupe constitué de 1, 2, 3 et 4 ;
R¹², R¹³, R¹⁴ sont, indépendamment les uns des autres, choisis dans le groupe constitué de H, CN, alkyle ayant de 1 à 8 atomes de carbone, aryle ayant de 6 à 10 atomes de carbone, et hétéroaryle ayant de 4 à 10 atomes de carbone.

6. Matériau photoactif selon l'une quelconque des revendications 1 à 5, dans lequel, dans la formule (I) X¹, X² sont, indépendamment l'un de l'autre, choisis dans le groupe constitué de H, F et Cl ; et/ou dans lequel Y¹, Y² sont, indépendamment l'un de l'autre, choisis dans le groupe constitué de O et S.

7. Matériau photoactif selon l'une quelconque des revendications 1 à 6, dans lequel, dans la formule (I) A et B sont, indépendamment l'un de l'autre, choisis dans le groupe constitué de phényle, phényle substitué, naphtyle, naphtyle substitué, biphényle, biphényle substitué, terphényle, terphényle substitué, anthranyle, anthranyle substitué, pyrényle, pyrényle substitué, pérylényle, pérylényle substitué, thiényle, thiényle substitué, pyridyle, pyridyle substitué, quinoléinyle, quinoléinyle substitué, bithiényle, bithiényle substitué, terthiényle, terthiényle substitué, dans lequel un substituant quelconque, s'il est présent, est un substituant Z², où chaque Z² est, indépendamment des autres, choisi dans le groupe constitué d'alkyle ayant 1 à 12 atomes de carbone, O(alkyle) ayant 1 à 6 atomes de carbone, F, Cl, CN, dicyanovinyle, cyanopyridylvinyle, cyanophénylvinyle, phényle, naphtyle, thiényle, bithiényle et pyridyle.

8. Matériau photoactif selon l'une quelconque des revendications 1 à 7, le matériau photoactif comprenant en outre un ou plusieurs matériau(x) semi-conducteur(s).

9. Matériau photoactif selon l'une quelconque des revendications 1 à 8, dans lequel la substance donneuse comprend ou est constituée d'un ou plusieurs composés de formule (I) tels que définis dans l'une quelconque des revendications 1 à 8, et dans lequel la substance accepteuse comprend ou est constituée d'un ou plusieurs composés choisis dans le groupe constitué de
(i) fullerènes et dérivés de fullerène, de préférence choisis dans le groupe constitué de C₆₀, C₇₀ et ester méthylique d'acide [6,6]-phényl-C₆₁-butyrique, et
(ii) 3,4,9,10-perylènetétracarboxyl-bisbenzimidazole (PTCBI).

10. Cellule solaire organique ou photodétecteur **caractérisé en ce qu'**il comprend un matériau photoactif selon l'une quelconque des revendications 1 à 9.

11. Dispositif de conversion photoélectrique comprenant ou constitué de deux ou plus de deux cellules solaires organiques selon la revendication 10.

12. Dispositif de conversion photoélectrique selon la revendication 11, dans lequel les cellules solaires organiques sont agencées en tant que cellules en tandem ou en tant que cellules en tandem inversé.

13. Composé de formule (I) tel que défini dans l'une quelconque des revendications 1 à 7 pour utilisation en tant que substance donneuse ou en tant que substance accepteuse dans un matériau photoactif, dans lequel le matériau photoactif fait partie d'une couche d'une bicouche donneur-accepteur ou partie d'une couche mixte donneur-accepteur (23 - Figure 3, 24 - Figure 4).

14. Utilisation d'un composé de formule (I) tel que défini dans l'une quelconque des revendications 1 à 7 en tant que substance donneuse ou en tant que substance accepteuse dans un matériau photoactif, dans laquelle le matériau photoactif fait partie d'une couche d'une bicouche donneur-accepteur ou partie d'une couche mixte donneur-accepteur (23 - Figure 3, 24 - Figure 4).
